# EUROPEAN PATENT SPECIFICATION

(11) **EP 4 301 452 B1**
(45) Date of publication and mention of the grant of the patent: **04.03.2026**
(21) Application number: 21709893.8
(22) Date of filing: 04.03.2021
(51) Int. Cl.: A61N 1/36

(54) **SYSTEM FOR GENERATING ELECTROMAGNETIC TREATMENT PROTOCOL BASED ON READINGS OF BIOPHYSICAL SIGNALS**
SYSTEM ZUR ERZEUGUNG EINES ELEKTROMAGNETISCHEN BEHANDLUNGSPROTOKOLLS AUF BASIS VON MESSWERTEN BIOPHYSIKALISCHER SIGNALE
SYSTÈME POUR GÉNÉRER UN PROTOCOLE DE TRAITEMENT ÉLECTROMAGNÉTIQUE SUR LA BASE DE LECTURES DE SIGNAUX BIOPHYSIQUES

(43) Date of publication of application: 10.01.2024
(73) Proprietor: EMHANCE d.o.o., 42000 Varazdin (HR)
(72) Inventor: Isakovic, Jasmina, 42000 Varazdin (HR)
(74) Representative: Sucic, Tatjana
(86) International application number: PCT/EP2021/025090
(87) International publication number: WO 2022/184225

(56) References cited:
- US-A- 4 754 753
- US-A1- 2010 114 212
- US-A1- 2019 358 465
- US-A9- 2017 348 048

## Description

### \FIELD OF THE INVENTION

The present invention relates to an electromagnetic treatment of the nervous system, other tissue or any organ affected by injury, inflammation or ischemia, including infectious diseases, deficiency diseases, hereditary diseases (including both genetic diseases and non-genetic hereditary diseases), and physiological diseases as well as other disorders. More particularly, the present invention relates to a system for generating electromagnetic treatment protocol concurrently with reading of biophysical signals.

### BACKGROUND OF THE INVENTION

Electromagnetic field (EMF) is a vector field created by the displacement of electrically charged objects where energy interactions with other electric charges or electromagnetic fields occur. Whilst electric field describes the space within which an electric charge exerts an electric force upon another charged body in its vicinity, a magnetic field is a field within which a charge will experience a magnetic influence.

According to their nature or type and direction, electromagnetic fields are divided into homogeneous and inhomogeneous. Homogeneous fields are characterized by spatial consistency and inhomogeneous fields are variable in space. If there is a change in the nature of the field in time, the field is called a time-varying field. Not only do these fields then interact with charges in their vicinity, but they can also influence each other. Just as a changing magnetic field induces an electric field, a changing electric field induces a magnetic field.

As a result of ongoing degeneration and demyelination within the nervous system, or inflammation in other regions of the body, many ion channels undergo changes in their activation kinetics and their distribution along the cell membrane, resulting in an imbalance of an ion flow and extra- or intra-cellular concentration. Since one of the main properties of electromagnetic fields is their ability to influence charged objects in their vicinity, EMFs can modify charge distribution along the cell membrane. They can also direct movement of ions and control the activity of voltage-gated ion channels. The innate charge on all the cells within the central nervous system makes them susceptible to electromagnetic field influence. Currently, the most common methods of electromagnetic field administration to the brain tissue consist of vagal nerve stimulation (VNS), (repetitive) transcranial magnetic stimulation (rTMS), deep brain stimulation (DBS), pulsed electromagnetic fields (PEMFs) and low-frequency magnetic fields (LF-MF), all of which, in one way or another, impact the signal transmission through neurons and their activity.

As such, application of time-varying electromagnetic fields targeted to the nervous system has been demonstrated as facilitating restoration of function after trauma, stroke, and spinal cord injury as well as manage pain and decrease inflammation. This application also shows potential for future use to treat other conditions of the brain or the nervous system, such as autism, Alzheimer's disease, epilepsy, Parkinson's disease, attention deficit hyperactivity disorder (ADHD), or any other health conditions.

In order to diagnose diseases, as well as track disease progression and success of therapy, various techniques have been developed for measuring activity of the brain and nervous system. For example, electroencephalography (EEG) may be utilized to measure overall activity of the brain. Magnetoencephalography (MEG) may be used to map local brain activity near the outer surface of the brain. Functional magnetic resonance imaging (fMRI) may acquire both spatial and temporal images of brain activity.

Document US2017348048 discloses methods and apparatuses for nerve modulation techniques such as ablation of nerve tissue or other destructive modulation technique through the walls of blood vessels and monitoring thereof. The apparatuses of US2017348048 do not provide a storage space configured to store a protocol and positioning map and that emitters and sensors operate as pairs, wherein the field delivered through each emitter is modified until it is achieved that the measured biophysical signal by a paired sensor is being within the reference electromagnetic field range. PCT publication WO2018047164 discloses a system that provides images of a patient's brain concurrently with performing cognitive tasks and imaging with EEG, MEG and fMRI technologies, and after having the patient's brain scanned digitally, it includes the generation of a treatment protocol by comparing a digital image of the patient's brain with one or more images of brain of other patients. The treatment is then set based on previous diagnoses. WO2018047164 focuses on creating the digital image of a patient's brain and, by comparing inactive parts of other patients' images, concluding which part of the brain needs to be stimulated. Furthermore, the system of WO2018047164 focuses on generation of bulk spatial maps of neural activity in order to generate a treatment protocol for input into an electromagnetic field generator, all for the purpose of treating the indicated impaired functionality using designated treatment frequencies. Since this system generates therapeutic electromagnetic field to treat dysfunction of neural networks, it appears to deliver systemic treatment - impacting both functional regions of the brain as well as those with impaired functionality. While WO2018047164 consists of a communication interface for receiving information containing data collected from a series of neural activity sensors located on the patient during a session of applied stimuli and a processor configured to generate multiple different treatment protocols, the system of the present invention consists of a portable unit with built in sensors, functioning on a feedback loop with emitters, for the purpose of therapy personalization during application itself, and a stationary unit for therapy design, treatment monitoring and calibration.

US publication US20170087367 uses MEG, EEG, tMRI technologies, PET scan, SPECT, ECOG, sMRI, OTT, MRS and fNRIS to digitally visualize the appearance of the brain and diagnose the region of the brain with functional deficits.

The system for delivering therapy consists of a housing for placing at least one electrode relative to the area of tissue, wherein the housing defines a cavity or channel such that the opening defined through the at least one electrode is open to the cavity or channel. This system functions on the principle of "ramp up/ramp down" method for gradually increasing the strength of the emitted field chosen from one of the stimulation modes from the group of transcranial magnetic stimulation (TMS), repetitive TMS (rTMS), pulsating electromagnetic fields (PEMF), transcranial alternating current stimulation (TACS), transcranial random noise stimulation (TRNS), time-varying electrical stimulation (TVES), and ultrasound brain stimulation (UBS).

One of the disadvantages of the aforementioned disclosures is planning a therapy protocol that is then kept constant during the singular duration of treatment, not accounting for the fact that the human body is a dynamic system, with ever-changing properties and states. As such, applying a constant therapy protocol for the time duration is not only disregarding the properties of human tissue in a real-time, as well as the organism's response, but is also less effective than a system of the present invention.

Another notable difference between a system of the present invention, and most of the prior art disclosures is that most of prior art focuses on improving cognitive properties of the patient after some trauma or illness, aiming at functional changes in nerves or neurons, while the system and method of the present invention uses electromagnetic fields of varied nature (such as pulsating inhomogeneous time-varying electromagnetic fields) that are similar to the fields emitted by the patient's brain or other tissue to restore cellular function through instigation of histological changes, not only in neurons but also in other cell types, for patients with diseases of the nervous system such as Alzheimer's, Multiple Sclerosis, stroke, ALS and any other autoimmune or other pathological conditions of the organism, as well as cancer. As opposed to only serving as signal amplification tool, the electromagnetic fields of the present invention may also be used to impact a variety of cellular processes (from cell death, activity, migration, division etc.) in different cell types such as those of the connective tissue, neural tissue, immunological system, muscle tissue etc., or organs in general and cause changes on the cellular level. The solution of the present invention works by inducing a reorientation of molecules within the cells, causing a deformation of embedded ion channels and altering their activation kinetics, as well as reorganizing the distribution of ions around the cellular membrane and within the intra- and extracellular space. This leads to cellular polarization, changing of the rate of ion and ligand binding, amplifying or diminishing the signal propagation as well as electromagnetic induction. Additionally, the system of the present invention utilize a paired feedback loop between the emitters and sensors in order to provide personalized treatment in accordance with real-time sensor readings.

Another distinct difference between the state-of-the-art disclosures and the technical solution of the present invention is a predefined range of electromagnetic field strength and frequency specifically selected as an optimal one for each cell type within all major tissue and organs for the purpose of a therapy optimization. The solution of the present invention takes into account a heterogeneity of the tissue and, while designing treatment protocols, defines a predefined range of electromagnetic field strength and frequency that should be used for optimal therapy effects on each organ or tissue, depending on the prevalent or targeted cell type. Since each cell within the human body has a natural resonant frequency of its membrane channels, targeting cells with these frequencies can increase or decrease their activity through causing a significant change of the ion flux across the cell membrane channels, inducing alteration in their cellular function. This could be especially important in treating autoimmune disorders such as Multiple Sclerosis where an increased activation of cells of the immune system causes demyelination, as well as cancer and other diseases and disorders where an increase in cellular activity may be noted, leading to ongoing pathological events.

As such, the technical solution of the present invention significantly improves previous systems as it includes pairing each of sensors and emitters during therapy application and a real-time monitoring of biological, chemical and physical parameters of the patient for the purpose of concurrently modifying treatment application as well as instigating changes in a variety of cellular processes (such as cell death, activity, migration, division etc.). Additionally, the present system is designed as such that can be used in conjunction with cell transplantation in order to serve the purpose of electromagnetic guidance of transplanted cells to the targeted location or a guided drug delivery system.

### SUMMARY OF THE INVENTION

There is thus provided, in accordance with an embodiment of the present invention, a system for generating and applying electromagnetic therapy protocol concurrently with reading of biological and/or biophysical and/or biochemical signals. Wherein all future mentions of the terms as used herein refers to "biophysical", "biological" or "biochemical" signals correspond to any or all of the aforementioned signal types.

The system for generating electromagnetic therapy protocol has a twofold purpose, the application of electromagnetic field and the measurement of electromagnetic fields, tissue impedance, resistance and other parameters to monitor the success and course of applied therapy in a real time. The system of the present invention is directed to generating focused electromagnetic fields affecting cells in certain areas of the brain, or any part of the body, and works on the principle of a feedback loop control system, where the nature, frequency and intensity of applied electromagnetic fields is automatically modified to optimize the course of applied therapy.

The main object of the present invention is to provide a protocol generation and treatment system for delivering a focused electromagnetic stimulation to a brain, nervous system, or any part of the body of a patient, concurrently with measurements of biological and/or biophysical and/or biochemical signals.

A protocol generation and treatment system of the present invention comprising: a main database configured to store at least one patient personal folder comprising a medical patient data, a plurality of baseline treatment protocols, each baseline treatment protocol being associated with a particular disease or an indicated impaired functionality of the nervous system, organ or other tissue affected by injury, inflammation or ischemia, a reference range of electromagnetic field strength and frequency providing optimal therapy effects on each organ or tissue, depending on the prevalent or targeted cell type and one or more associated reference sensors and emitters positioning maps, and representative neural tissue, organs and tissues profiles associated with reference electromagnetic field ranges for each neural tissue, organ and cell type of an healthy individuals; an machine learning software configured to compare the medical patient data associated with the particular disease or the indicated impaired functionality, design and extrapolate one or more baseline treatment protocols associated with the particular disease or the indicated impaired functionality, to provide one or more reference sensors and emitters positioning maps associated to said baseline treatment protocols and to determine which of said positioning maps and baseline treatment protocols are suitable for the patient; a portable apparatus comprising an electromagnetic field generator configured to generate electromagnetic fields; an array of sensors configured to measure biophysical signals and an array of emitters configured to deliver electromagnetic fields; a computing unit configured to control the electromagnetic field generator, for recording in a real-time measured biophysical signal from each of the array of sensors, comparing measured biophysical signal with the reference electromagnetic field range and to deliver a plurality of different electromagnetic fields through the array of emitters and a power supply for components of the portable apparatus; and a stationary device configured to calibrate the portable apparatus, test the array of sensors and emitters for their accuracy, design the baseline treatment protocol and to save the baseline treatment protocol and reference sensors and emitters positioning map in the main database and a storage space of the portable apparatus before patient use, and to remotely control operation of the portable apparatus.

Herein, the term "suitable for the patient" is defined as a set of mandatory and nonessential conditions that must be met in order for a treatment protocol or placement map to be chosen. If the treatment protocol or the placement map has been obtained from a medical patient data within the database, the set of mandatory conditions that have to be met consists of: disease, organ or tissue affected, stage of disease progression or duration of symptoms, and a patient age. Nonessential conditions include, but are not limited to, a region of the organ affected, medical history or results of previous diagnostic readings and current symptoms. In case no treatment protocol that satisfies the aforementioned conditions exists, the machine learning algorithm may pull reference electromagnetic field ranges for each tissue or organ, as well as baseline placement maps, and uses that as a baseline treatment protocol.

The computing unit is further configured to modify electromagnetic field delivered through each of the array of emitters concurrently with measurement of a biophysical signal from each of the array of sensors, wherein each of the array of emitters and each of the array of sensors positioned in a close proximity to each other are operating as a pair, wherein the electromagnetic field delivered through each emitter is continuously modified until it is achieved that the measured biological and/or biophysical and/or biochemical signal by a paired sensor is being within the reference electromagnetic field or a signal range. The baseline treatment protocol may be modified until the sensor measures an electromagnetic field within the reference electromagnetic field range for that specific tissue or organ, or other biophysical signal within its reference range. Wherein this process of modifying the baseline treatment protocol would entail creating a personalized treatment protocol. In such cases where no activity profile can be measured that corresponds to reference electromagnetic field ranges, or reference ranges of other measurements, the baseline treatment protocol may be stopped, and the patient might report to a health institution or clinic for a check in. This can be followed by a generation of new placement maps, or reprogramming of the sensors and emitters to continue with a conservative treatment protocol during which the sensors start recording the response patterns of tissue during applied treatment. In cases where the cellular activity patterns show no change over time, the baseline treatment protocol may be adjusted to emit electromagnetic fields within the reference electromagnetic field range, taking into account a natural resonant frequency of specific cell types, in order to cause electromagnetic induction in a surrounding tissue and achieve synchronization of a cellular activity with the reference electromagnetic ranges. Additionally, new sensor and emitter placement maps may be generated and a baseline treatment protocol might be followed until changes in sensor readings are observed.

Furthermore, in accordance with an embodiment of the present invention, a generated baseline or personalized treatment protocol defines a characteristic of the applied electromagnetic field selected from a group of characteristics consisting of amplitude, frequency, strength, direction, duration of application and type.

Furthermore, in accordance with an embodiment of the present invention, a generated baseline or personalized treatment protocol comprising a reference range of electromagnetic field strength and frequency that should be used for optimal therapy effects on each organ or tissue, depending on the prevalent or targeted cell type.

Furthermore, in accordance with an embodiment of the present invention, an applied electromagnetic field may be a constant or time-varying homogenous and/or inhomogeneous electromagnetic field, or a combination thereof.

Furthermore, in accordance with an embodiment of the present invention, there is provided a portable apparatus.

Furthermore, in accordance with an embodiment of the present invention, a computing unit of the portable apparatus is configured to modify electromagnetic field delivered through each of an array of emitters concurrently with measurement of a biophysical signal from each of an array of sensors, wherein each of the array of emitters and each of the array of sensors positioned in a close proximity to each other are operating as a pair.

Further, in accordance with an embodiment of the present invention, a method of generating a baseline treatment protocol is provided.

Further, in accordance with an embodiment of the present invention, a method of generating a personalized baseline treatment protocol is provided.

Further, in accordance with an embodiment of the present invention, a method for generating reference sensors and emitters placement map is provided.

### BRIEF DESCRIPTION OF THE DRAWINGS

The details of various examples are set forth in the accompanying drawings and the description below. Other features, objects, and advantages will be apparent from the description and drawings, and from the claims.
Fig. 1 schematically illustrates a perspective view of a portable apparatus of a system for generating electromagnetic treatment protocol, in accordance with an embodiment of the present invention.
Fig. 2 schematically illustrates a perspective view of a stationary device of a system, in accordance with an embodiment of the present invention.
Fig. 3A schematically illustrates a perspective, side and top view of an emitter, in accordance with an embodiment of the present invention.
Fig. 3B schematically illustrates a side view of an emitter having different shapes of electrodes that may be contained within a housing of said emitter, in accordance with embodiments of the present invention.
Fig. 4 schematically illustrates front, back and top view of the most common variations in electrodes and sensors positioning on a patient.
Fig. 5 illustrates a portable apparatus for delivering an electromagnetic field therapy to a patient to manage an infectious disease, in accordance with an embodiment of the present invention.
Fig. 6 illustrates a portable apparatus for use in a vaccine production, in accordance with an embodiment of the present invention.
Fig. 7 illustrates a portable apparatus for use to stimulate an immune system to respond to a vaccine more rapidly and vigorously, as a form of an electromagnetic vaccine adjuvant, in accordance with an embodiment of the present invention.
Fig. 8 is another embodiment of a system for generating electromagnetic treatment protocol arranged in a hospital environment, in accordance with an embodiment of the present invention.
Fig. 9 is a flowchart depicting a method of generating a baseline electromagnetic treatment protocol for treating a nervous system, other tissue or any organ affected by injury, inflammation or ischemia.
Fig. 10 is a flowchart depicting a method of generating a personalized baseline electromagnetic treatment protocol considering biophysical signals and calibrating a portable apparatus to suit a patient's needs.
Fig. 11 is a flowchart depicting a method of treating disorders and diseases of the nervous system, other tissue or any organ affected by injury, inflammation or ischemia in an individual.
Fig. 12 is a flowchart depicting a method of generating one or more reference sensors and emitters placement maps.
Fig. 13 is a diagram illustrating a mean intensity of glial fibrillary acidic protein in an experimental condition.
Fig. 14 is a diagram illustrating a mean intensity of beta tubulin in an experimental condition.
Fig. 15 is a diagram illustrating a mean intensity of nestin in an experimental condition.

### DETAILED DESCRIPTION OF THE INVENTION

In the following detailed description, numerous specific details are set forth in order to provide a thorough understanding of the invention. However, it will be understood by those of ordinary skill in the art that the invention may be practiced without these specific details. In other instances, well-known methods, procedures, components, apparatuses, modules, units and/or circuits have not been described in detail so as not to obscure the invention.

Although embodiments of the invention are not limited in this regard, discussions utilizing terms such as, for example, "processing," "computing," "calculating," "determining," "establishing", "analyzing", "checking", "computerized neural networks", "machine learning", "deep learning", "signal processing", or the like, may refer to operation(s) and/or process(es) of a computer, a computing unit, a computing platform, a computing system, or other electronic computing device, that manipulates and/or transforms data represented as physical (e.g., electronic) quantities within the computer's registers and/or memories into other data similarly represented as physical quantities within the computer's registers and/or memories or other information non- transitory storage medium (e.g., a memory) that may store instructions to perform operations and/or processes. Although embodiments of the invention are not limited in this regard, the terms "plurality" "a plurality", "array" and "an array" as used herein may include, for example, "multiple" or "two or more". The terms "plurality" or "a plurality" may be used throughout the specification to describe two or more components, apparatuses, elements, units, parameters, or the like. Unless explicitly stated, the method embodiments described herein are not constrained to a particular order or sequence. Additionally, some of the described method embodiments or elements thereof can occur or be performed simultaneously, at the same point in time, or concurrently. Unless otherwise indicated, the conjunction "or" as used herein is to be understood as inclusive (any or all of the stated options).

Some embodiments of the invention may include an article such as a computer, computing unit or processor readable medium, or a computer or processor non-transitory storage medium, such as for example a memory, a disk drive, a USB flash memory, or any other type of storage medium which may or may not be at a well-defined location (such as cloud storage), encoding, including or storing instructions, e.g., computer-executable instructions, which when executed by a processor, computation unit or controller, carry out methods disclosed herein.

The methods and system described herein provide for characterizing readings of biophysical signals of a nervous system or other tissue/organ affected by injury, inflammation, ischemia, chronic condition or degeneration and, based on readings of biophysical signals, concurrently setting electromagnetic field parameters in a real time for each area of the nervous system or other tissue affected by injury, inflammation, ischemia, chronic condition or degeneration.

As used herein the term "electromagnetic field" or "electromagnetic fields" refers to a constant or time-varying homogenous and/or inhomogeneous electromagnetic field(s) characterized by its strength, direction, duration of application, frequency, amplitude and type.

Main objectives of the present invention are following:
- the system works on the principle of personalized therapy by imitating the electromagnetic properties (amplitude, frequency, wavelength or intensity) or other biological, biophysical or biochemical parameters of the brain or the nervous system, other tissue or any organ through information obtained from multiple sensors and biomarkers on the target part of the patient, as well as assisting with neuroimaging for deeper regions of the human body;
- replication and application of time-varying pulsating homogeneous and/or inhomogeneous electromagnetic fields;
- reading the properties and parameters of the signal obtained from the nervous system, other tissue or any organ in a real time, and applying an identical or drastically different signal as read from the patient's nervous system, other tissue or any organ;
- measurement of electromagnetic fields (EMFs) using sensors that may be SQUID sensors, and other sensors that measure physiological and electro-physiological data, tissue properties and other properties of other cells before and concurrently with therapy application;
- an apparatus may be portable and can be worn by the patient for several hours every day;
- measurement of electromagnetic fields when patients perform cognitive tasks, or any other passive activity, to better determine where in the nervous system, other tissue or any organ the deficiency occurs and, based on the signals received from the sensor, focusing treatment on the part of the nervous system, other tissue or any organ in which there is a deficiency;
- using a built-in feedback loop between paired sensors and emitters, wherein treatment protocol is automatically modified during its application; and
- combining electromagnetic field treatment and cell implantation through electromagnetic conduction and/or guidance of stem cells or other cells within the tissue; isolating the target cell from the patient's blood/plasma/cerebrospinal fluid and culturing them in cell culture and then applying EMF to design a personalized therapy protocol i.e., to determine the optimal strength and frequency required for electromagnetic field application.

In accordance with an embodiment of the present invention, a protocol for treatment by constant or time-varying homogenous and/or inhomogeneous electromagnetic fields is automatically generated by a system. The treatment protocol may be inputted into a treatment system, the treatment system includes an apparatus for generating an oscillating time-varying homogenous and/or inhomogeneous electromagnetic field that may be applied to the entire body or a location on the body of the patient. (Reference herein to an electromagnetic refers to a time- varying magnetic field that is generated by an electromagnet or an electromagnetic field generator configured to generate a time-varying pulsating electromagnetic field including its strength, direction, frequency, amplitude and type.) The oscillating electromagnetic field is characterized by a frequency and amplitude, type, strength or intensity. As used herein the terms "intensity" and "amplitude" are used interchangeably to represent an amplitude, maximum, average, root mean square, or other characteristic or representative value indicative of the strength of an applied electromagnetic field or characterizing a spectrum of a measured neural or other tissue/organ activity signal. A treatment protocol may define the frequency, amplitude, intensity and strength of the applied electromagnetic field, as well as one or more other characteristics of the electromagnetic field (e.g., duration of each application of the field, type of the electromagnetic field such as homogenous or inhomogeneous field, number of applications, interval between applications, or other characteristics). A treatment protocol may define two or more electromagnetic fields of different frequencies, intensities and types that may be applied to the patient in a defined sequence (e.g., concurrently or at different times).

The treatment system may be configured with an interface for receiving information regarding a patient. The information may include clinical data with a description that describes one or more impaired functionalities of the patient as well as any other comorbidities. In particular, the description may describe impaired functionality that is known or suspected to be related to impaired function of the patient's nervous system or other tissue affected by injury, inflammation or ischemia. For example, the clinical data may be input by a medical professional based on examination of the patient, or based on the patient's statements as well as his previous medical history.

In addition, the received information includes results of measurements of function of the patient's nervous system at various points of the nervous system (e.g., by sensors placed at various locations on the patient's skin, or elsewhere). The measurements may include an array of sensors that are arranged at known locations relative to the patient's brain, spinal cord, other components of the patient's nervous system or other tissue affected by injury, inflammation or ischemia. For example, the sensors may include sensors that are designed for electroencephalography (EEG), magnetoencephalography (MEG), electromyoneurography (EMNG) or for other types of sensors designed to measure physiological and electro-physiological data, the properties of tissues, other cells and organs. According to preferred embodiment of the invention, sensors include impedance sensors, temperature sensors, magnetic field sensors (such as SQUID sensors or Hall effect sensors) and electric field or impulse sensors. Such information from sensors may be input directly to a communication module, machine learning software or the main database for storage and analysis, or input by a user, e.g., as text or as medical codes (e.g., selected from a menu).

Sensor measurements may be acquired from the patient concurrently with a therapy session that includes successive application of one or more stimulus. As used herein, a stimulus may be applied actively by the patient. For example, the patient may perform a voluntary physical task (e.g., rest state, moving of a limb or body part, resisting movement of a limb or body part, speaking, controlling breathing, looking at an object, or another active physical task), cognitive (e.g., rest state, imagining a situation, thinking about a particular subject, attempting to solve a problem, concentrating on selected sensory input, or another active cognitive task), attempting to perform a physical task (e.g., concentrating on moving a limb that is paralyzed, has been amputated, or that is restrained), or another voluntary or active task. A stimulus may be applied by an outside agent during which time the patient remains passive. For example, a limb or body part of the patient may be moved by an outside agent (e.g., by another person or by a machine), the patient may be subjected to sensory input, or may otherwise be passively stimulated. A stimulus may include observing an action performed by another person (e.g., in person or recorded), or an animated action on a screen, virtual reality device, or otherwise. Application of a stimulus may include concurrent (or alternating) application of two or more different stimuli. Active or passive application of a stimulus is interchangeably referred to herein as performance of a task.

The sensor measurement data may be analyzed to obtain a set of processed data (e.g., electromagnetic field amplitude, frequency, intensity and strength, temperature, physiological and electro-physiological data and properties of tissues and other cells such as the electrical impedance, permittivity or magnetic permeability of specific tissue types or organs). For example, each set of data may represent a measurement by a sensor (or, in some cases, by a group of two or more sensors) during application of a particular stimulus or plurality of stimuli. Alternatively, or in addition, measurements that were acquired during application of a particular stimulus may be marked or labeled with an identification of that stimulus.

In some cases, the analysis may include distinguishing relevant parameters based on statistical analysis using Al, machine learning or deep learning software. For example, such machine learning may identify correlations between features of one or more measured electromagnetic fields and application of a particular electromagnetic field in a particular area of the patient's body. Such analysis may be based on current or previously acquired patient data, or on analysis of a group of patients, e.g., that share similar clinical characteristics, e.g., are characterized by common features of their clinical histories.

One or more measured biophysical signals that are relevant to or that may be associated with an indicated impaired functionality or pathological events of the nervous system or other tissue affected by injury, inflammation or ischemia of the patient may be identified. For example, the impaired functionality may be identified or suspected based on received clinical data (e.g., from symptoms that were reported by the patient or the patient's family or associates, that were noted during examination or observation by a medical professional, that are obtained by a medical test, or otherwise). Pathological events may be identified or suspected based on received sensor measurements or performed pathological analysis.

The measured neural activity, or electrical activity of cells within other tissue, at each identified electromagnetic field may be organized to generate a spatial activity map. For example, the spatial position of a measured electromagnetic field may be determined by the identity of the sensors by which that electromagnetic field was measured. The spatial map may show the electromagnetic field of neural activity, or electrical activity of other cells, at each mapped location. As used herein, the spatial position may refer to a location on or within the patient's body, or to the location of a particular sensor. The mapped neural activity, or activity of other cell types, may be displayed in the form of the spatial activity map or otherwise (e.g., as a set of coordinates or sensor identifiers and a particular result), and may include a value or a correlation between two values, or one or more quantities, that are derived from one or more brain wave spectra.

One or more corresponding reference baseline treatment protocols and corresponding one or more reference sensors and emitters positioning maps may be retrieved from a main database or other data storage facility. The reference baseline treatment protocols and said positioning maps may be retrieved on the basis of any or all of the following parameters, including but not limited to the same disease or condition, symptoms and tissue or organ affected. Each baseline treatment protocol, as well as sensors and emitters positioning map, may show a representative electromagnetic field, or values of other biophysical, biochemical or biological parameters, for either a healthy nervous system or other tissue (e.g., based on measurements on subjects whose nervous systems or other tissues were determined to be functioning without impairment) or for a nervous system or other tissue affected by injury, inflammation or ischemia whose function is defective in a known manner as well as a reference range of electromagnetic field strength and frequency that should be used for optimal therapy effects on each organ or tissue, depending on the prevalent or targeted cell type.

Each of the retrieved reference sensors and emitters positioning maps may be compared with the corresponding (on the basis of a readings of biophysical signals of a nervous system or other tissue affected by injury, inflammation or ischemia) generated sensors and emitters positioning map. Comparison of regions of calculated variations in the electromagnetic field and skin impedance with respect to the reference sensors and emitters positioning maps may indicate regions where the generated sensors and emitters positioning map is indicative of defective function or an ongoing pathological event. For example, one or more locations in the comparison between the reference sensors and emitters positioning map and the generated sensors and emitters positioning map may indicate a level of function at a spatial region that deviates from a reference sensors and emitters positioning map that is representative of a healthy nervous system, or a level of function at a spatial region that is similar to a reference sensors and emitters positioning map that is representative of a defectively functioning neural network.

When one or more locations on one or more generated sensors and emitters positioning map are indicative of defective function, a treatment protocol may be generated. The treatment protocol may be input into a portable apparatus for generating a therapeutic electromagnetic field. The treatment protocol may specify characteristics of one or more time-varying therapeutic electromagnetic fields that are to be applied to the patient. The treatment protocol may be entered to the electromagnetic field generator automatically, e.g., by a computer, or may be selected or entered by a user, e.g., based on the baseline or personalized treatment protocol. The treatment protocol, when input into the electromagnetic field generator, may cause the electromagnetic field generator to generate a sequence of a time-varying homogenous and/or inhomogeneous electromagnetic field(s) characterized by its strength, direction, frequency, amplitude and type, wherein for each organ and cell type a reference electromagnetic field range has been predefined. The treatment protocol may specify other characteristics of the sequence of fields. These characteristics may include one or more of field intensities or amplitudes, durations, an order of application of the fields of different frequencies, interval between applications of the different fields, number of applications of each of the fields, or other parameters. The treatment protocol may be applied to the entire body of the patient, or to a part of the body of the patient, such as one or more of the head, neck and spinal cord or other tissue.

Each identified electromagnetic field may be associated with a particular neural network or other tissue. As used herein, a neural network includes components of the nervous system that are associated with a particular functionality of the nervous system. Typically, response to a particular stimulus or performance of a particular task may involve two or more different neural networks.

Reference electromagnetic field ranges and patients skin impedances determined for each connective tissue, neural tissue, immunological system, muscle tissue and organ may have been previously derived from analysis of measurements that were made on a population of subjects, in some cases including the patient being currently treated.

For detecting a location of trauma in other parts of the nervous system, skin impedance sensors may be used with which different types of hematomas in all parts of the patient's nervous system, organ or other tissue may be detected.

For example, the population may have included both healthy individuals whose nervous system appeared to be fully functional, and individuals whose nervous systems were determined to be functioning defectively in known ways, and in some cases, subjects who appear to be fully functional but may have deficiency in one or more neural networks (e.g., whose other neural networks compensate for the deficiency). Comparisons among the various measurements may indicate the properties of electromagnetic field and skin impedance that are associated which each functionality, and thus with each neural network or other tissue. Spatial mapping over the nervous system (e.g., of one or more of brain, spinal cord, or other major nerves) of nervous system or other tissue activity may indicate regions of the nervous system or tissue where deviation in neural activity is associated with a particular defect in function of a particular neural network or other tissue.

A neural activity profile may be based on neural function measurements that are made on a person or a population. The neural activity profile characterizes the neural activity of a person (patient, person in control group, or other person) or population (e.g., after application of averaging or other statistical manipulation to measurements made on individuals in the population). The neural activity profile may be based on EEG, MEG, or other measurements that are made on the brain or other parts of the nervous system of one or more human subjects. For example, sensors similar to MEG sensors, or similar to EEG sensors or other sensors, may be used to measure neural activity in the brain, spinal cord, or in other parts of the nervous system. As used herein, an MEG or EEG measurement refers a measurement any part of the nervous system using an MEG-like or EEG-like sensor, respectively. Similarly, an MEG or EEG sensor, as used herein, refers to a sensor that functions similarly to an MEG (sensing magnetic fields) or EEG (sensing electrical current or voltage) sensor, whether the sensor is intended for measurement of brain activity or activity of another component of the nervous system.

The measurements, in particular EEG, MEG, EMNG or similar measurements, may be interpreted to yield a map of measured neural activity at a plurality of identifiable locations in the nervous system. For example, EEG or MEG sensors or other local sensors may be placed at a set of predetermined locations on persons head, back, another location where neural activity occurs (e.g., in the vicinity of an active organ or limb, sense organ, or elsewhere), or at any combination of the above, in order to obtain a spatial map of neural activity. A location of the measured local neural activity may be derived from the locations of the sensor or sensor that measured the local activity. In some cases, where the local neural activity is measured by a plurality of sensors, triangulation or other techniques may be applied to determine a location of the measured local activity relative to the known locations of the sensors. In addition, measurements (e.g., functional magnetic resonance imaging (fMRI) or other measurements) may be interpreted to indicate whole brain activity.

The measurements may be made as the nervous system of each subject is activating a particular neural function. For example, a neural function may be associated with the human subject performing one or more tasks. Tasks may include resting (e.g., for the purpose of establishing one or more baseline measurements of neural activity to which other measured neural function may be compared), performing one or more active (e.g., a voluntary movement of a limb, facial feature, or other body part by the patient) or passive (e.g., a part of the body being moved by another person, a machine, or other external agent) motor activities or movements, performing one or more active or passive cognitive tasks (e.g., object recognition, memory retrieval, problem solving, or another cognitive activity), automatic task (e.g., being subjected to an environmental condition, sensory input, or other stimulus that activates the autonomous nervous system), or other tasks. Components of the nervous system where activity is altered from a baseline level during performance of a task are herein referred to as a neural network that is associated with the task and neural function.

A main database of representative neural activity profiles and profiles of other tissue or organ activity comprising reference electromagnetic field ranges for each organ and cell type may be generated by performing measurements on a population of subjects. Each reference electromagnetic field range is determined by frequency, amplitude, intensity and strength. The population of subjects may include individuals whose nervous systems, or other organs and tissue in question, are determined to be healthy (e.g., as determined by a lack of known defects in or trauma to their nervous systems or other tissues) and are determined to be healthy in general. In some cases, the population may be divided into subpopulations (e.g., divided by age, sex, or other characteristics that are known or suspected to affect a neural activity profile). A neural activity profile and profiles of other tissue or organ activity is determined for a population that is assumed to be healthy is referred to herein as a reference profile.

In some cases, defective function of a neural network or other tissue of a patient may be suspected. For example, such defective function may be suspected following stroke, trauma, illness, disorder or another occurrence that may sometimes result in, or that may be suspected of resulting in, injury to one or more components of the nervous system or other tissue. As another example, the patient may report having difficulty, or may be observed to have difficulty, in successfully performing one or more tasks such that defective functioning of a neural network may be suspected.

When defective functioning of a neural network is suspected, a series of neural activity measurements may be made on the patient. For example, MEG, EEG, EMNG or other measurements of neural activity may be made on the patient as the patient performs a series of tasks. The series of tasks may include a complete set of tasks that are used in obtaining a complete neural activity profile of the patient. Alternatively, the performed tasks may be limited to a subset of the tasks that are related to a particular suspected neural network defect or that would be effective in expediting diagnosis of a defect in a particular neural network.

The acquired patient neural activity profile may then be compared with a reference profile. The reference profile may include a standard profile for all individuals, or may be characteristic of a subpopulation. For example, the reference profile may be specific to one or more of a particular age group, gender, racial or ethnic group, professional or academic background, or an otherwise defined subpopulation. In some cases, a comparison may be limited to those frequencies that are characteristic of or are suspected to be related to a particular suspected defect. For example, the treatment system may be configured to retrieve the electromagnetic fields that have been determined to be characteristic of one or more neural networks, or electrical activity of other groups of cells. The comparison may then check whether measured neural activity differs at one of the identified characteristic electromagnetic fields from a reference neural activity profile, or electrical activity profile of other cells, that is retrieved from a main database comprising reference profiles. For example, the comparison may indicate that at one or more locations, a spectrum of neural activity that is associated with a particular neural network as measured for the patient may differ from the reference neural activity profile for that neural network at those locations. The differences may be quantified. For example, a difference score for each neural network may be a function of differences between the measured neural activity profile and a corresponding reference neural activity profile. For example, the difference score may be expressed as a fraction or percent of a normal neural activity for each neural network. Same difference scores may be obtained for the electrical or other biological activity of other cells within the affected tissue or organ.

The comparison may yield a one or more suggested treatment protocols for an electromagnetic treatment to correct any identified defects in one or more neural networks or other tissue. For example, the treatment protocol may specify a frequency and strength or frequency spectrum and strength spectrum of a therapeutic time varying electromagnetic field that may be applied to the patient. The treatment protocol may specify a location on the patient to which the specified therapeutic electromagnetic field is to be applied, or may specify that the field is to be applied to the entire brain, to a particular body part, or to the entire body of the patient. The treatment protocol may specify an amplitude of the therapeutic electromagnetic field, or another quantity related to the strength of the therapeutic electromagnetic field that is to be applied. The treatment protocol may specify type (e.g., homogenous and/or inhomogeneous) of the therapeutic electromagnetic field that is to be applied. The treatment protocol may specify duration of exposure to the therapeutic electromagnetic field. The treatment protocol may specify a number of treatment sessions, an interval between subsequent treatment sessions, or otherwise define a series of treatments. Where treatment is required for two or more neural networks or tissues, the treatment protocol may specify application of treatment (e.g., at two or more field frequencies) for the different neural networks and tissues. For example, the treatment protocol may specify an order of treatment, e.g., an order of application of electromagnetic fields having different type of said fields or intensities, an interval between successive application of the different electromagnetic fields, or other parameters related to treatment of different neural networks or tissues.

The treatment protocol may specify a result of a monitored biological, biophysical or biochemical sensors readings that may be indicative of the efficacy of the treatment. During a treatment session, the treatment protocol may then be automatically modified in accordance, and concurrently with, the monitored biological, biophysical or biochemical sensors readings. A treatment system, or a treatment monitoring unit of a protocol generation system, may include one or more sensors whose measurements may be indicative of activity in a neural network and other tissue or in one or more parts of the nervous system or other organs. For example, monitored biophysical sensor readings may include monitored activity in a region of the brain or the nervous system or other tissue affected by injury, inflammation or ischemia as indicated by electromagnetic field, tissue impedance, the temperature and skin conductivity of that region (e.g., where an increase in local temperature is indicative of increased local blood flow, in turn indicative of increased local brain activity). Alternatively, or in addition, another sensor may be used. Such other sensors may include, for example, electrocardiogram (ECG) sensors, EEG or MEG sensors, EMNG sensors for electrical measurement of muscle activity, near infrared sensors, blood content analyzers, or one or more other types of sensors. The sensors may include one or more sensors that are configured to measure motion of the patient's body. For example, such motion sensors may include a remote motion sensor (e.g., based on ultrasound, electromagnetic waves or pulses, or otherwise), a virtual reality (VR) sensor (e.g., a VR glove, full or partial VR suit, or other VR sensor), light emitting elements or reflectors attached to the patient and recorded by one or more imaging devices, or another type of motion sensor. Sensors that are configured for placement on or near the patient, e.g., within the applied therapeutic electromagnetic field, may be configured for operation within magnetic fields (e.g., designed for use during with magnetic resonance imaging (MRI), or on a patient who is located within the magnetic field of an MRI machine). Alternatively, or in addition, the treatment system may be configured to apply the therapeutic field intermittently (e.g., with periodic breaks during which no field is applied), with the sensors configured only during breaks between operation of the fields.

For example, a baseline treatment protocol may indicate a duration, frequency, type and a range of an applied reference electromagnetic field that is based on representative results, theory, throughout preclinical and clinical testing or otherwise on a typical anticipated response to treatment. However, the response may vary from individual patient to individual patient. Therefore, during application of the treatment electromagnetic field, a temperature map, activity map or other monitoring result of the patient's head or nervous system may be acquired, e.g., using a thermal camera or other sensors. For example, a measured temperature may be indicative of whether activity has increased as a result of the treatment in a region of the nervous system that is associated with the neural network that is being treated (e.g., in a part of the brain where activity may be anticipated to increase as a result of the treatment due to recovery of the neural network in response to stimulation by the applied field).

A treatment system may be configured to adjust or modify a baseline treatment protocol in accordance with one or more monitored biophysical sensor readings using the built-in feedback loop system. For example, if a monitored electromagnetic field has increased to a level that is indicative of improved function of a neural network or other tissue, treatment with an electromagnetic field that is characteristic of that neural network or tissue may be stopped (e.g., for the remainder of a current treatment session, or otherwise) or reduced (e.g., reducing amplitude, frequency, intensity or duration). As such, the treatment protocol may be modified until the sensor measures an electromagnetic field within the reference electromagnetic field range for that tissue or organ, or other biophysical signal within its reference range. In such cases where no activity profile is measured corresponding to reference electromagnetic field ranges, or reference ranges of other measurements, the treatment protocol may be stopped, and the patient might report to the health institution or clinic for a check in. This may be followed by a generation of new placement maps or reprogramming of the sensors and emitters to continue with a conservative treatment protocol during which the sensors start recording the response patterns of tissue during applied treatment. In cases where the cellular activity patterns show no change over time, the treatment protocol may be adjusted to emit electromagnetic fields within the reference electromagnetic field range, taking into account the natural resonant frequency of specific cell types, in order to cause electromagnetic induction in the surrounding tissue and achieve synchronization of cellular activity with the reference electromagnetic field ranges. Additionally, new sensor and emitter placement maps may be generated and the baseline treatment protocol might be followed until changes in sensor readings are observed.

Application of the therapeutic time varying inhomogeneous electromagnetic field may facilitate regeneration or functional restoration of the neural network or other organs or tissue. Inhomogeneous electromagnetic fields affect the interaction between cells as well the interaction between the cells and the extracellular matrix, the interaction of cells with nerve fibers, and affect the nature of the fields around neurons. Because they are similar, in nature, to innate electromagnetic fields within the human body, inhomogeneous electromagnetic fields reduce negative components of the inflammatory response responsible for neuronal damage, and damage to other tissue or organ, and promote tissue regeneration. Through causing deformation of embedded ion channels, altering their activation kinetics and reorganizing of the ion distribution around the cellular membrane and within the intra- and extracellular space, inhomogeneous electromagnetic fields can control the opening and closing of voltage gated ion channels, change the rate of ion and ligand binding as well as cause electromagnetic induction. This, in turn, changes the dynamics of cellular activation, migration, activity and division. Alternatively, these fields can also decrease cellular activity and division in those cases where they are causing a disruption in the organism's functioning and contribute to an ongoing pathological event.

The difference scores for each neural network, or other cells of interest, may be presented to an operator of the system. A physician or other healthcare professional may examine the difference scores. In some cases, the protocol generation system may be configured to automatically determine a baseline treatment protocol. In some cases, the protocol generation system may be configured to automatically transmit a baseline treatment protocol to a treatment system. The treatment system may be configured (e.g., when a human operator indicates that treatment is to be applied to the patient for which the baseline treatment protocol was generated) to generate one or more electromagnetic fields in accordance with the transmitted protocol.

The system healthcare professional may then determine whether or not treatment is required, and may prioritize application of one or more of the treatment protocols suggested by the system, for treatment of one or more neural networks or other tissues. The determination of the treatment protocol by the healthcare professional may be based on, in addition to the initial clinical examination of the patient, patient preference, extent of damage to each neural network or tissue, patient history, time limitations, or other information or criteria. Determination of an order of treatment of different neural networks or other tissues may take into consideration such factors as the patient's needs and preferences, estimated time required to treat each neural network, need for treatment (e.g., as indicated by a difference score in combination with other considerations), past success, or other considerations.

The system of the present invention may be used for combining electromagnetic field therapy in combination with stem cell implantation to provoke an electromagnetic conduction of stem cells or other cells within tissues; isolating the target cell from the patient's blood / plasma / cerebrospinal fluid and culturing in cell culture and then applying electromagnetic field therapy to design a personalized therapy, i.e. to determine the optimal strength and frequency to stimulate / regenerate cells in certain areas of the brain or any body part or tissue. The system's magnetic properties can also be used to guide labeled cells to the desired location within the body, i.e., magnetic guidance of stem cells, or other cells meant for transplantation, in order to enable their differentiation into other cell types of targeted organs or tissue in order to initiate tissue regeneration through release of growth factors or functionally replace the impaired cells. Furthermore, the system can be used to enable targeted drug therapy through electromagnetic guidance of the drug towards the desired location, as well as facilitate its faster release and action.

A stationary device, in general, is placed in specialized institutions (hospitals, clinics, etc.) through which trained medical staff could configure therapies for patients, calibrate a portable apparatus as well as remotely control the operation of a portable apparatus that the patient could take home, in order to prevent misuse of the device by the user.

Another possible embodiment of the present invention is to install a wall with a plurality of electrodes on the inside that may generate specialized electromagnetic therapy in which staff / patients would go for a short period of time to combat infectious diseases that occur in hospitals (during operations, pandemics, etc.) as well as provide a general boost of the immune system for those that are immunologically compromised or otherwise have an autoimmune disease.

Cancer or tumor treatment therapy for the patient can also be achieved with the system of the present invention by acting with electromagnetic fields of the same frequency or inverse sign from those previously measured from the cancer cells themselves through our sensors (SQUID / Hall Effect etc.), with the aim of destroying / weakening tumor or cancer cells through disrupting their division, impairing their activity as well as enhancing their recognition by the cells of the immune system through upregulation of MHC antigen processing and presentation, as well as its expression. Also, such application may be used in combination with radiotherapy to improve the impact of radiation, reduce a number of negative consequences and serve as a kind of mitigation of the harmful effects of radiotherapy in the patient. Moreover, this system could also be used in conjunction with chemotherapy in order to improve the patient's wellbeing as well as enhance the workings of his immune system.

Electromagnetic fields may be used to modify cellular signals and factors relevant to the activation of danger signals within cells of the human body, especially those within our immune system. They have the ability to induce a reorientation of molecules within the cells, causing deformation of embedded ion channels and altering their activation kinetics and, with that, decreasing or increasing cellular activity. Electromagnetic fields can also cause reorganization of ion distribution around the cellular membrane and within the intra- and extracellular space, leading to cellular polarization as well as controlling of the opening and closing of voltage gated channels. They can also change the rate of ion and ligand binding, amplifying or diminishing the signal propagation as well as cause electromagnetic induction, i.e., additional current flow at specific regions of the body where the fields have not been applied, further amplifying the cellular signaling cascade. On top of this, they are also a non-invasive modality, more economical and safer than drugs or surgery and may reach a larger number of patients and health professionals.

The two main mechanisms of action of electromagnetic field are the modulation of cell adhesion through a change in the external charge on the cell and changing the rate and nature of cellular signaling and communication, both that between cells themselves as well as between cells and the extracellular matrix. Depending on the frequency and nature of the field in question, the target cells can trigger an anti-inflammatory or an inflammatory response, or completely suppress their response.

By acting on the major histocompatibility complex II (MHC II) and major histocompatibility complex I (MHC I) as well as heat shock proteins (HSP), adenosine triphosphate (ATP), interleukins and other molecular mediators, electromagnetic fields can improve antigen recognition by immune cells and slow or accelerate the immune response, as well as initiate tissue regeneration. Interestingly, as recent studies suggest, some damage to the lungs of patients with COVID-19 could also be caused by an overly active immune response, so suppressing the immune response by targeting it with specific field frequencies could also be crucial. In this case, system of the present inventior may be used as a stand-alone therapy to alleviate the symptoms of this disease, or any other infectious disease, or in combination with a developed vaccine or medicine used to relieve symptoms, or transplant stem cells or any other cells. Further, the system of the present invention may cause inhibition of the inflammatory response by reducing levels of proinflammatory cytokines as well as reducing the recruitment of inflammatory cells to the lungs or other organs that have been affected by an overactive immune response. Moreover, when a stem cell transplant is performed prior to the administration of electromagnetic field, the system of the present invention may not only be used to reduce the proinflammatory immune response, but its magnetic properties can be used to direct labeled stem cells to the desired site in order to cause functional regeneration of the impacted organ.

One or more coils may be operated to generate one or more time-varying electromagnetic fields in accordance with a selected treatment protocol. The electromagnetic fields may be applied to a selected section of the body (e.g., all or part of the head, torso, thorax, abdominal area, a limb, or another section of the body), or may be applied to the patient's entire body.

By measuring a tissue impedance via an impedance sensor, the site of inflammation / deficiency in the tissue may be more quickly and accurately determined, and information from that sensor can help detect different types of hematomas on a patient's tissue.

A treatment protocol generation system is configured to generate a baseline treatment protocol for a patient, wherein said protocol may be updated in a real-time concurrently with readings of biophysical signals, yielding a personalized baseline treatment protocol. The treatment protocol generation system may be separate from a system that includes treatment system and that is configured to apply a therapeutic electromagnetic field to patient. In some cases, the protocol generation system may be configured to communicate with the separate system that includes treatment system. In some cases, the protocol generation system may be incorporated in a single protocol generation and treatment system that includes treatment system or protocol generation system may incorporate treatment system. The protocol generation system includes, or may communicate with, an array of sensors 500. The array of sensors 500 may include one or more types of sensors that are configured to measure one or more types of neural activity, temperature, tissue impedance, permeability and permittivity, and electromagnetic fields, as well as a multitude of other biophysical, biological or biochemical parameters.

The protocol generation and treatment system based on concurrent readings of biophysical signals comprises:
a main database configured to store at least one patient personal folder comprising a medical patient data, a plurality of baseline treatment protocols, each baseline treatment protocol including one or more associated reference sensors 500 and emitters 400 positioning maps, each baseline treatment protocol is associated with a particular disease or disorder, or an indicated impaired functionality of the nervous system, organ or other tissue affected by injury,
inflammation or ischemia of a patient, each baseline treatment protocol is associated with the reference range of electromagnetic field strength and frequency provided for optimal therapy effects on each organ or tissue, depending on the prevalent or targeted cell type,
an machine learning software and a processor configured to compare the medical patient data with that from similar baseline treatment protocols, extrapolate one or more baseline treatment protocols associated to the particular disease or the indicated impaired functionality, to provide one or more reference sensors 500 and emitters 400 positioning maps associated to the baseline treatment protocols and to check which of the positioning maps and the baseline treatment protocols is suitable for the patient, considering readings of biophysical signals, and to modify the positioning map and the baseline treatment protocol considering readings of biophysical signals into personalized positioning maps and treatment protocols,
a portable apparatus 100 comprising an electromagnetic field generator configured to generate a constant or time-varying pulsating electromagnetic field including its strength, direction, frequency, amplitude and type, a power supply for components of the portable apparatus 100, an array of sensors 500 configured to measure biological, biophysical and biochemical signals including electromagnetic fields and an array of emitters 400 configured to deliver electromagnetic fields, a computing unit 104 configured for recording readings of biophysical signals from each of the array of sensors 500, compare readings of biophysical signals with the reference electromagnetic field ranges to identify one or more of the pluralities of biophysical signals corresponding to the particular disease or the indicated impaired functionality, wherein each baseline treatment protocol defines characteristics of a time-varying pulsating electromagnetic field to be applied through each of the array of emitters 400,
a stationary device 200 comprising a machine learning software, a processor and a communication interface, a stationary device 200 is configured to calibrate the portable apparatus 100, test the array of sensors 500 and emitters 400 for their accuracy, save the baseline treatment protocol in a storage space of the portable apparatus 100 before patient use, store information comprising data collected from an array of neural activity sensors placed on a patient during a session of applied stimuli and data collected from the array of sensors 500 that were placed on the patient during treatment application.

The treatment protocol generation method may be executed by the computing unit 104. The treatment protocol generation method may be executed when the array of sensors 500 are positioned to measure electromagnetic fields, temperature, patient's skin conductivity. The acquired measured biophysical signals may include data for different regions of the nervous system, any tissue or organ and while the patient is performing different tasks or is subjected to a session of different applied stimuli.

The acquired measured biophysical signals may be analyzed to calculate an electromagnetic field range of one or more measurements of acquired recorded biophysical signals. For example, each measured biophysical signal that is detected by each sensor 500 (or by a group of two or more sensors) may be spectrally analyzed to yield a set of electromagnetic fields, e.g., each characterizing neural activity of a neural network, or electrical activity of any other cell types, at a location as measured by a sensor 500 or by group of sensors 500 (at a single or at neighboring locations, along a single nerve, or otherwise expected to produce a signal that typifies a location within the nervous system). A separate electromagnetic field may be calculated for each applied stimulus and for each sensor 500 location.

The treatment system may then be operated in accordance with the baseline treatment protocol to cause the electromagnetic field generator to generate the reference electromagnetic field range for each organ/cell type.

The baseline treatment protocol may be generated by selecting a previously designed treatment protocol from a main database of previously designed baseline treatment protocols. Each baseline treatment protocol is associated one or more reference sensors 500 and emitters 400 positioning maps as well as the reference range of electromagnetic field strength and frequency provided for optimal therapy effects on each organ or tissue, depending on the prevalent or targeted cell type. As another example, the treatment protocol may be generated on the basis of the identified subset, as well as on other factors (e.g., as derived from clinical data). For example, an order of application of the different types of electromagnetic fields, or a relative duration of treatment of each neural network, tissue or organ, may be determined by an urgency of treatment of each neural network, tissue or organ based on seriousness of the deviation from healthy function, on patient requirements or preferences, or on other criteria.

In some cases, more than one baseline treatment protocol may be generated. For example, several different baseline treatment protocols may be generated from which a medical professional may select one for application in treatment and one or more reference sensors 500 and emitters 400 positioning maps. After obtaining measurements from the biophysical signals from the array of sensors 500, a medical professional may select one reference sensors 500 and emitters 400 positioning map. The generated baseline treatment protocol may be made available for use in the portable apparatus 100. The generated baseline treatment protocol may be made available for use to a location on the body of the patient, or to the entire body of the patient.

The baseline treatment protocol may specify a sequence of applied electromagnetic field. Each applied electromagnetic field may be characterized by one or more frequency, nature, duration, and amplitude, or by other characteristics. The baseline treatment protocol may indicate an order of application of different electromagnetic fields, frequency of application, interval between successive applications, or other characteristics. Each baseline treatment protocol may be associated with evaluation that may be considered by a healthcare professional in choosing between two or more suggested baseline treatment protocols. A selected baseline treatment protocol may be applied (e.g., automatically or manually) in operating portable apparatus 100 to treat a patient. Application of the baseline treatment protocol may be modified during application of a therapeutic electromagnetic field to the patient. For example, the patient may be monitored during application of the treatment via one or more monitoring sensors or the array of sensors 500. Application of the therapeutic electromagnetic field of each of the array of emitters 400 may be modified (e.g., by changing the duration, amplitude, frequency, nature or other characteristic of the applied field) in accordance with a resulting measurement of the biophysical signals from each of the array of sensors 500, wherein each particular emitter 400 is positioned in a close proximity to each particular sensor 500, and said emitter 400 and sensor 500 are operating as a pair.

A treatment protocol generation method may be repeated after application of a therapeutic electromagnetic field. For example, the treatment protocol generation method may be re-executed when efficacy of treatment is to be evaluated.

The portable apparatus 100 is operative to obtain measurements of biophysical, biochemical or other biological signals from each of the array of sensors 500 concurrently with applying constant or time-varying pulsating electromagnetic field through each of the array of emitters 400, wherein the computing unit 104 is further configured to modify, in a real time, the time-varying pulsating electromagnetic field of each of the array of emitters 400 positioned in a particular area of a patient's body based on measurements from each of the array of sensors 500 positioned near each of the array of emitters 400, wherein the type, strength, frequency, direction and intensity of the time-varying pulsating electromagnetic field of each of the array of emitters 400 is modified to deliver at least a reference electromagnetic field in a range being characteristic to the particular area of the patient body of an healthy individual. Therefore, each of the array of sensors 500 positioned near the each of the array of emitters 400 are configured as a pair operating by a feedback loop control system. When the nature of the measured electromagnetic field around the neuron, other tissue or organ changes significantly or some abnormalities are noticed, portable apparatus 100 will automatically reduce, increase or completely stop the delivery of electromagnetic field of all or some of the arrays of emitters 400. The baseline treatment protocol may be constantly modified concurrently with readings of biophysical signals from each of the array of sensors 500. Therefore, each update of the baseline treatment protocol becomes a personalized treatment protocol.

The time-varying pulsating electromagnetic field of each of the array of emitters 400 may be set to deliver the reference electromagnet field being characteristic to the particular area of the patient body of the healthy individual or may be set to deliver electromagnet field matching to obtained measurements from each of the array of sensors 500, applying a drastically different signal which was measured from the patients tissue/organ or applying an electromagnetic field characterized by a different waveform (as opposed to the one being measured by the sensors 500) in order to cause destructive or partially destructive interference. In this case, the waveform of the applied signal has to be different from the one measured by the sensors 500 and, through changing the applied electromagnetic field's frequency and amplitude, partially or fully destructive interference may be achieved.

Drastically "different signal" refers to a signal having a different amplitude, frequency, intensity and/or a different waveform. The term "waveform" refers to periodic waveform types (sine, square, triangle, sawtooth, ramp up, ramp down, square, pulse, rounded pulse, circular pulse, triangular pulse, ramp pulse, sine cubed, flame, semicircle wave and all the variations of these waveforms), inverse waveform types of the ones mentioned above (such as arcsine, arctangent, arccosine and reverse square, reverse triangle, reverse sawtooth, etc. waves) and random waveform types that are not necessarily periodic.

When applying a "different waveform", a reverse/ inverse waveform of that which is being read by the sensors may be applied, or a completely different waveform, from the list above, may be applied (e.g. reading sine but applying tan or square). If the goal is to accomplish some form of constructive or destructive interference, then the portable apparatus 100 works with the variations of the same waveform type using the actual waveform (constructive interference) or its inverse (destructive interference). For example, if the shape of the acquired signal is sine, and the goal is to achieve constructive interference, the electromagnetic field whose waveform is sine shaped would be applied. On the other hand, if destructive interference were to be achieved, then an arcsine waveform would be applied, since it is the inverse waveform of the sine - and so on for each waveform. If the read signal is a square wave, constructive interference is obtained by applying a square wave, and destructive interference is obtained if a reverse square wave is applied.

Partially destructive or partially constructive interference may be obtained if the same, or opposite, waveform of a different amplitude or frequency is applied. Furthermore, depending on the suggestions of a machine learning software, completely different waveforms to the initial ones (sine - square) may be applied if the goal is not to achieve a specific value of interference.

The type of the time-varying pulsating electromagnetic field delivered through each of the array of emitters 400 may be homogenous and/or inhomogeneous.

Neural activity sensors may be incorporated into the stationary device 200. For example, the neural activity sensors may be located in a hospital, clinic, rehabilitation center, or within an open environment, or may be portable or transportable to a location of the patient. Neural activity sensors may also be incorporated into the portable apparatus 100 within the array of sensors 500.

For example, neural activity sensors may include an array of MEG sensors. Each MEG sensor may be configured to measure a magnetic field that results from electrical currents that are created by neural activity in the brain. Typically, each MEG sensor measures magnetic fields that originate in a region of the brain that is close to that MEG sensor. Thus, measurements by an array of MEG sensors may yield a spatial map of neural activity in the brain.

Neural activity sensors may include EEG electrodes. EEG electrodes may be configured for attachment to the scalp of patient, e.g., at standard locations on the scalp. EEG electrodes may be configured to measure electrical potential at on the scalp. The measured electrical potentials may be indicative of electrical activity within a section of the brain. Thus, measurements by EEG electrodes may yield a measurement of neural activity in the section of the brain near which EEG electrodes are attached.

Neural activity sensors may include EMNG electrodes. EMNG electrodes measure the speed and conductivity of motor and sensory nerves using a sterile needle electrode or with an electrode which is stimulated with a current. Quality of measured muscle responses may tell us about a severity of a damaged tissue and a type of damage of peripheral nerves.

Neural activity sensors may include spinal neural activity sensors. For example, each spinal neural activity sensor may be identical to, or operate similarly to, MEG sensors. In this case, an array of spinal neural activity sensors arranged along the spine of patient may measure magnetic fields that result from electrical currents that are created by neural activity in the spinal cord. Thus, measurements using spinal neural activity sensors may yield a map of neural activity within the spinal cord. In some cases, spinal neural activity sensors may be operating similarly to EEG electrodes, or in another manner suitable for measuring neural activity in the spinal cord. In some cases, spinal neural activity sensors, or other types of sensors, may be configured for placement near, and measurement of, neural activity in other parts of the nervous system (e.g., near one or more nerves).

Neural activity sensors may include other types of sensors for measurement of neural activity, or activity of any other cell type within the tissue affected by injury, inflammation or ischemia for the purpose of determining of a correct medical diagnosis.

According to preferred embodiment of the invention, the array of sensors 500 include impedance sensors, temperature sensors, electric and magnetic field sensors (such as SQUID sensors and/or Hall effect sensors) as well as permittivity and permeability sensors. The extreme sensitivity of SQUIDs makes them ideal for studies in biology. Magnetoencephalography (MEG), for example, uses measurements from an array of SQUIDs to make inferences about neural activity inside brains. Because SQUIDs can operate at acquisition rates much higher than the highest temporal frequency of interest in the signals emitted by the brain (kHz), MEG achieves good temporal resolution. A novel application of SQUIDs is the magnetic marker monitoring method, which is used to trace the path of orally applied drugs. In the clinical environment SQUIDs are used in cardiology for magnetic field imaging (MFI), which detects the magnetic field of the heart for diagnosis and risk stratification. A Hall-effect sensor (or simply Hall sensor) is a device to measure the magnitude of a magnetic field. Its output voltage is directly proportional to the magnetic field strength through it.

Generally, arrangement of the array of sensors 500 and the array of emitters 400 may be set to match readings of biophysical signals that indicate the area of the nervous system or other tissue affected by injury, inflammation or ischemia. If, after a number of treatment sessions, readings of biophysical signals of the area of the nervous system or other tissue affected by injury, inflammation or ischemia mostly affected by neurodegeneration or inflammation show improvement in clinical outcomes, the machine learning software may order a re-positioning of sensors 500 and emitters 400 (i.e. new sensors 500 and emitters 400 positioning map) concurrently with setting of a new baseline treatment protocol for other areas of the patient body that still show indicated impaired functionality of the nervous system or other tissue/organ affected by injury, inflammation or ischemia. Said new baseline treatment protocol in accordance with said repositioning map may also be constantly updated in real-time concurrently with readings of biophysical signals from each of the array of sensors 500.

According to the preferred embodiment of the invention, electromagnetic fields emitted by each of the array of emitters 400 may be modified in a real-time concurrently with readings of biophysical signals from each of the array of sensors 500, wherein each particular emitter 400 is positioned in a close proximity to each particular sensor 500, said emitter 400 and sensor 500 are operating as a pair.

The portable apparatus 100 may include a helmet, cap, or other headgear or arrangement for placement on or around the head of patient wherein the array of sensors 500 and the array of emitters 400 may be movable so that the time-varying pulsating electromagnetic field may be delivered to an area of brain affected by neurodegeneration, ischemia, injury or inflammation. More preferably, arrangement of the array of sensors 500 and the array of emitters 400 may be set to match readings of biophysical signals that indicate the area of brain, or other tissue, mostly affected by neurodegeneration, ischemia, injury or inflammation. If, after the number of treatment sessions, readings of biophysical signals of the area of brain show improvement in clinical outcomes, arrangement of the array of sensors 500 and the array of emitters 400 may be changed to other areas of brain, or other tissue, also affected by neurodegeneration, ischemia, injury or inflammation.

After the placement map with the position of the sensors 500 and emitters 400 has been made by the machine learning software, the electrodes and emitters may be placed onto a custom-made enclosure for the patient's tissue/ organ. When the machine learning software calculates the exact coordinates for an optimal position of the array of sensors 500 and emitters 400, additionally an extender may be made which will be able to easily attach to the main enclosure, providing structural stability, and will have the ability to precisely mount a sensor 500 or emitter 400 to it. Said enclosure may be provided with a casing having adjustable pre-installed array of sensors 500 and emitters 400 which may then be moved along X, Y and Z-axis to an optimal treatment position.

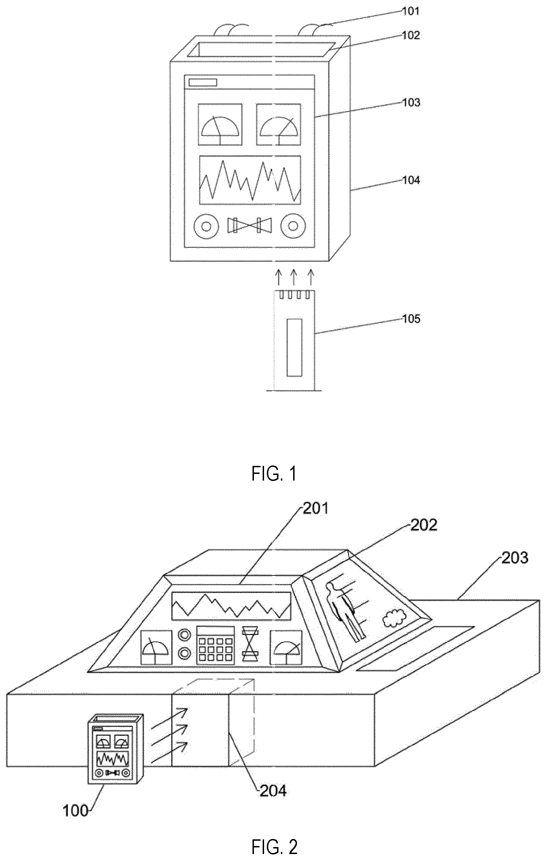

Fig. 1 illustrates schematically a perspective view of the portable apparatus 100 of the system of the present invention. Generally, it is designed as the portable apparatus 100 with adjustable straps 101 such that it may be carried on the back or shoulders of patients in order to provide with treatment throughout the day or the time of wear. It consists of a basic pocket 102 providing a storage area for the array of sensors 500 and the array of emitters 400, a touchscreen display 103 for indicating a type of the therapy protocol, time left, status of a battery, general information about previous and current therapy sessions (current, voltage and frequency used, graphical depictions of readings of biophysical signals etc.), and all other important information, whilst enabling the modifying of therapy protocol concurrently with readings of biophysical signals. The portable unit 100 is also configured to warn patients about improper use or its functioning. The touchscreen display 103 is configured to enable concurrent modification of the therapy protocol, via cloud services and through the processor and computing unit 104, considering readings of biophysical signals from the array of sensors 500. The touchscreen display 103 is configured to show the strength of electromagnetic fields around neurons or other tissue and its change during the therapy session. The touchscreen display 103 is wirelessly connected, via a portable part of the portable apparatus 100, to an online main database which can be accessed in real time by the patient's family medical professional and monitor progress of a delivered therapy protocol outcome and, if necessary, perform and emergency interventions.

Within itself, the portable apparatus 100 houses the computing unit 104 for recording all the measurements of biophysical signals from the array of sensors 500 and then concurrently delivering the baseline or personalized treatment protocol via each of the array of emitters 400 being controlled by the computing unit 104 through a feedback loop control system. Finally, the portable apparatus 100 comprises the power supply such as a rechargeable battery 105 which may be inserted into said portable apparatus 100 for the purpose of maintaining its portability. The portable apparatus 100 of the system comprises a few main components on an inside such as, but not limited to, a main control board including processor, a RAM memory and a storage space which will control the machine learning software with a sensor/emitter section, a battery management system with the rechargeable battery 105, a security section which includes fuses and FID elements etc. The portable apparatus 100 is provided with multiple rechargeable batteries 105 so when the patient uses up one battery 105, he can put it to charge, and instead of waiting for it to charge, just continue his treatment session with the other previously fully charged one. The portable apparatus 100 may also be configured with a variety of solar panels to serve as a power source for recharging the battery.

The computing unit 104 may include one or more units that are configured to interface with the array of sensors 500 via touchscreen display 103. For example, units of the computing unit 104 may be enclosed in a single case or housing, or may be separate from one another. Separate units of the computing unit 104 may be interconnected by cables, or via a wireless connection. In some cases, units of the computing unit 104 may be remote from one another. When units of the computing unit 104 are remote from one another, different units of the computing unit 104 may be configured to communicate with one another via a network or other cable or a wireless communication channel. In some cases, part or all of touchscreen display 103 may be included in a processor (in which case, touchscreen display 103 may include only a connection of processor to the array of sensors 500). In some cases, part or all of the functionality of the touchscreen display 103 may be provided by the processor operating in accordance with programmed instructions.

The portable apparatus 100 may be connected to the stationary device 200 through a port arranged on a back side of the portable device 100, which will fit into a connection port 204 of the stationary device 200 resulting in the calibration and initialization process, or any other connection method that might be deemed more appropriate for the situation. After the initialization process is done, and the baseline or new treatment protocol has been checked by the machine learning software and supervised by medical professionals, the patient may take the portable apparatus 100 to his home and use it within the comfort of his home, instead of similar devices being used in the hospital, which is an environment many patient's dislike. In this way, treatment may be provided for longer periods of time, as well as be tailored to fit the patient's lifestyle and severity of symptoms, often necessitating longer treatment exposure times. The portable apparatus 100 may vary in its shape; constitutive elements and other components may be changed depending on the desired effect, patient's needs and duration of therapy.

FIG. 2 shows a perspective view of the stationary device 200 of the system of the present invention, a calibration, controlling and treatment station, usually positioned within health institutions. The stationary apparatus 200 comprises an automatic device calibration station 201 and a processing, controlling and treatment station 202 (hereinafter "the processing station 202"). Upon insertion of the portable apparatus 100 into the connection port 204 of the stationary device 200, the portable apparatus 100 may be automatically calibrated, the array of sensors 500 and emitters 400 tested for their accuracy and the baseline treatment protocol delivery before the portable apparatus 100 is handed to the patient for use. The main advantages of such automatic calibration are diminishing of the possible human error in the therapy planning process as well as application of machine learning algorithms in order to optimize the baseline therapy protocol and duration, based on previous cases of similar disease management and patient recovery outcome. On the other hand, the processing station 202 is configured to enable visualization of collected measurements from the array of sensors 500 available within the portable apparatus 100, visualization of other diagnostic tests done previously, patient's medical history as well as to plan future treatment protocols and track patient's recovery progress through personalized data representation, filtering and recording. This data can then be stored locally in the main database as well as on a cloud. The idea is for medical professionals to be able to use the workspace in front of LCD screens for a better interpretation and understanding of patient recovery progress. The portable apparatus 100 brought by the patient may be inserted into the connection port 204 for the calibration sequence to initiate, as well as enable medical professionals to make changes to the baseline treatment protocol and to inspect the patient recovery outcome while undergoing treatment. The computing unit 104 of the portable apparatus 100 is configured to send treatment data saved on its HDD or a cloud storage and to a stationary device storage for an easier access by the educated medical professional. The medical professional should pass an education course on how to operate both of the apparatuses 100 and 200, as well as the optimal way to cooperate with the machine learning software in a joint goal of adjusting the baseline treatment protocol based on recorded measurements of biophysical signals each time the patient undergoes a control health assessment. The stationary device 200 may be connected with a wire into the power outlet and in an ideal setup it would require to be connected to an emergency backup generator so when a power outage happens it wouldn't lose any potential information about patients' treatments as well as compromise the integrity of the stationary device 200 while the calibration process is in progress.

A communication interface of the processing station 202 may interface with one or more components. For example, the communication interface may communicate with an input device in order to enable operator input into the processing station 202. For example, operator input may include clinical data, operation commands for controlling operation of a component of protocol generation system (or of treatment system), or other input data. The communication interface may include a device or port for reading or writing to one or more types of portable or removable data storage media. For example, a data storage medium may be used to store acquired measurement data or clinical data for input into the processing station 202, or the treatment protocol for input into the treatment system.

Measured quantities may be stored as acquired measurement data on a data storage device. For example, acquired measurement data may be in the form of a sequence of sensed electromagnetic fields, tissue impedance, permittivity and permeability, temperature or other measurements that are acquired at known times. The processing station 202 may be configured to apply one or more data manipulation techniques to acquired measurement data. For example, data manipulation techniques may include applying averaging, digital noise reduction, applying a calibration, scaling, separating a signal from a background signal (e.g., a signal that is acquired when patient is resting or idle), smoothing or sharpening (e.g., applying a low pass or high pass digital filter), or another analysis technique.

One or more signal processing or other data analysis techniques may be applied to acquired measurement data. Application of the data analysis techniques may result in one or more reference profiles (neural activity profile and other tissue activity profile). For example, each neural network activity profile results from a data analysis that identifies one or more features that distinguish measured neural activity that is associated with a particular neural network (e.g., based on measurements that are acquired while patient is performing a task that is associated with that particular neural network) from measured neural activity that is associated with a different neural network (or when no neural network is expected to be active, such as when patient is at rest). The resulting reference profiles may be stored as part of the main database on the data storage device. For example, acquired measurement data in the form of a time sequence of measured electromagnetic fields may be processed to obtain a spectrum in the form of amplitude, frequency, intensity and strength. One or more transforms, such as a Fourier transform, wavelet transform, or other transform, may be applied to acquired measurement data. The transform may be applied by utilizing an algorithm such as, for example, the fast Fourier transform (FFT), filtered FFT, wavelet analysis, or another spectral filter. Other analytic techniques may be applied. Such other techniques may include one or more statistical evaluations such as application of one or more computational neural networks, machine learning, or deep learning algorithms.

The analysis may be performed separately for each of the arrays of sensors 500, for a subset of the arrays of sensors 500 (e.g., of the same type at neighboring or functionally linked locations), or for all sensors 500 (e.g., of a particular type). The analysis may identify a difference between an individual patient and expected performance. The analysis may identify whether an individual patient is associated with a more generalized group for which particular treatment protocols have been previously found to be beneficial.

When data is acquired for group of subjects whose nervous system is apparently healthy (e.g., the subjects' medical histories are free of any events that may be suspected to injure the nervous system, the subjects are free of any observable symptoms of injury to the nervous system, or are otherwise determined to be healthy), the acquired analyzed data may be combined, e.g., by averaging or by application of another statistical or data combination technique. The resulting combination of each profile may be stored in the main database as a reference profile for a neural network, organ or other tissue.

Similar measurements and analysis may be performed on subjects whose nervous system, organ or other tissue is known to function defectively in one or more ways. For example, the defective function may be related to performance of a particular task (e.g., motor, cognitive, sensory, or other task), or may be associated with a known anatomical abnormality (e.g., as determined by an imaging technique, or otherwise). A resulting profile may be compared with the corresponding representative reference profile. The comparison may yield one or more electromagnetic fields that are characteristic of a neural network, organ or tissue that is associated with the defective function.

The protocol generation system may measure a neural activity profile on a patient when injury to the patient's nervous system is suspected. Neural activity measurements at one or more locations on the body of patient may be acquired using neural activity sensors such as an array of electroencephalography (EEG), electromyoneurography (EMNG) or of magnetoencephalography (MEG) sensors and the array of sensors 500 such as temperature sensors, SQUID and/or Hall effect sensors or other sensors for measuring electromagnetic fields. Analysis of the measurements may result in a patient profile that may be compared with one or more reference profiles. In particular, when patient presents symptoms that are associated with defective function of a particular neural network, analysis may be limited to, or may prioritize or emphasize, comparison at neural network electromagnetic fields that are associated with that neural network.

The patient profile may be compared by the processing station 202 to a corresponding reference profile that may be retrieved from the main database. One or more neural network, organ or other tissue electromagnetic fields may be selected for analysis. The neural network, organ or other tissue electromagnetic fields may be selected on the basis of preclinical and clinical data. For example, a patient may present one or more symptoms that are recorded in clinical data. For example, a symptom may include inability or reduced ability to move a voluntarily limb (e.g., where the skeleton and musculature are intact), to speak, to receive a sensation, to perform a cognitive task (e.g., read, to identify a person or object, to solve a problem, or another cognitive task), or to respond to another type of stimulus. Each symptom could result from impaired function of one or more neural networks, organs or other tissue affected by injury, inflammation or ischemia (e.g., related to various memory, planning, motor activation, feedback, organization, or other functionality of the nervous system). Each selected value of the electromagnetic field may correspond to one of the neural networks. Further analysis then attempts to detect impaired function of one or more of the selected neural networks. Same respectively applies to any organ or other tissue.

In some cases, a comparison between a patient profile and a corresponding retrieved reference profile may detect one or more differences or deviations between function of a neural network, organ or other tissue in patient and function of that neural network, organ or other tissue in a healthy subject. The comparison may identify an electromagnetic field range at which a detected deviation occurs. Such a deviation may be indicative of an anomaly in the behavior of the corresponding neural network, specific cell group, organ or other tissue.

In particular, the comparison may indicate a deviation in amplitude, frequency, intensity or strength of the patient profile at one or more electromagnetic fields. Measured patient profile may be spatially mapped over the patient's brain, nervous system, organ or other tissue affected by injury, inflammation or ischemia.

The processing station 202 may be configured to generate a baseline treatment protocol based on detected differences between a patient profile and the retrieved reference profile. The generated baseline treatment protocol may be saved on a data storage device of the portable apparatus 100. Alternatively, or in addition, e.g., where protocol generation system and treatment system are incorporated into a single system or are configured to intercommunicate, the portable apparatus 100 may automatically retrieve the baseline treatment protocol from a data storage device of the treatment system to be used to treat the corresponding patient.

The generated baseline treatment protocol may be applied by computing unit 104 in operating electromagnetic field generator configured to generate an electric current to flow through treatment coils 304-310. Operation of the electromagnetic field generator may cause a current to flow through one or more electrically conductive treatment coils 304-310. The flow of current through treatment coils 304-310 may generate a therapeutic electromagnetic field that is applied to patient. An amplitude of the current flow may determine an amplitude of the therapeutic electromagnetic field. Application of the therapeutic electromagnetic field to patient in accordance with the baseline treatment protocol or a personalized treatment protocol may result in increased neural activity, or activity of surrounding cells within the target tissue. The increased neural activity, combined with increased activity of cells within the surrounding tissue, may facilitate rehabilitation processes in the nervous system and patient's body, and thus facilitate restoration of nervous system function and tissue regeneration.

The array of sensors 500 is used to monitor patient during operation of electromagnetic field generator. The array of sensors 500 is incorporated into the portable apparatus 100. In addition to SQUID, Hall effect sensors, impedance sensors and temperature sensors, the array of sensors 500 may include one or more sensors that are configured to measure motion of the patient's body, as well as tissue permittivity and permeability. The array of sensors 500 may be configured to not interfere with magnetic fields emitted by the array of emitters 400.

The computing unit 104 may be configured to modify or update, in a real-time, the application of the baseline treatment protocol (e.g., at least as applied to a specific patient) in accordance with one or more quantities that are monitored by the array of sensors 500. For example, if a value of a monitored quantity obtained by one or more sensors 500 deviates from an expected value, the computing unit 104 may be configured to change an amplitude, frequency, type or duration of a therapeutic electromagnetic field that is applied, via respective one or more emitters 400 each operating as a pair with said sensors 500, to patient in order to achieve the expected value. The expected value lies within an electromagnetic field range, or other biological, biophysical or biochemical signal range, for each organ and cell type that has been defined through preclinical and clinical testing. As another example, if the value of the monitored quantity is indicative of the treatment being complete (e.g., having had a desired or expected effect on patient), continued application of the therapeutic electromagnetic field may be curtailed, reduced, or otherwise modified.

In some cases, the baseline treatment protocol may indicate a period of time or duration of application of the therapeutic electromagnetic field. The period of time may be for a single treatment session (in which case the protocol may indicate one or more of a frequency of repetition of application of the therapeutic electromagnetic field, a number of times for applying the therapeutic electromagnetic field, and an interval between applications of the therapeutic electromagnetic field). The period of time may include an overall treatment time (e.g., in a situation where different applications of the therapeutic electromagnetic field are expected to have a cumulative effect). The baseline treatment protocol may include a repetition scheme for repeated application of treatments (e.g., for a particular patient or for a class or group of patients).

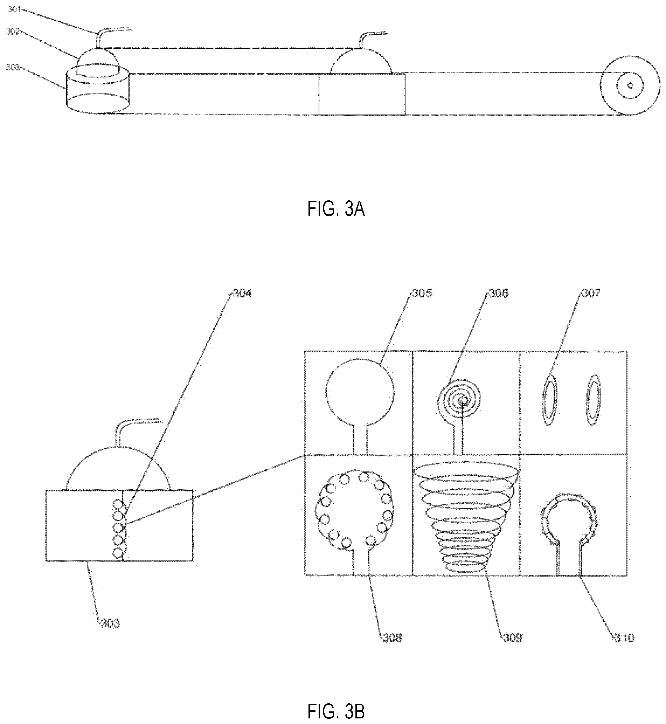

Fig. 3A schematically illustrates a perspective, side and top view of one emitter 400. In general, each emitter 400 comprises an emitter coil housing 303, electrodes 304-310 arranged within said housing 303, said electrodes 304-310 are configured to emit homogeneous and inhomogeneous time-varying or stationary electromagnetic fields, a housing cap 302 arranged at a top of the emitter coil housing 303 enabling easier coil extraction and replacement in order to obtain different electromagnetic field types and natures thereof, as well as a current-carrying wire 301 connected with the electrodes 304-310 in order to provide power supply to said electrodes.

Each of the emitters 400 would be positioned to the specific part of patient's body onto which a conductive gel or other conductive substance was previously applied in order to maximize the efficiency of therapy delivery. The emitter coil housing 303 is made of a non-magnetic material such as plastic, so that it does not interact with electromagnetic fields produced by each of the coils 304-310. Dimensions of the emitter coil housing 303 may vary in regards a specific area of the patient body that the array of emitters 400 would be applied to. Wires that supply power to the coils 304-310 are insulated so that it minimizes the risk of an accident while the portable apparatus 100 is being used and they are directly connected to the sensor/emitter part of the computing unit 104 inside of the portable apparatus 100. Each of the array of emitters coils 304-310 do not interfere with the array of sensors 500 for measuring biophysical signals particularly electromagnetic fields, since each of the array of emitters 400 comprise a built-in element (so-called screener) that filters higher intensity electromagnetic fields emitted by coils 304-310.

Each of the emitters 400 are connected to the computing unit 104 which regulates the power, amplitude, type and frequency of the electromagnetic fields as well as duration of treatment, defined by the baseline treatment protocol. The baseline treatment protocol is generated by the machine learning software and supervised by a medical professional. The baseline treatment protocol may be directly modified by virtue of a portable apparatus 100 settings.

According to the various embodiments of the invention, the emitter coil housing 303 may be altered based on preferences as well as to enable the emitter coil housing 303 comprising multiple coils 304-310 or contain a different configuration/arrangement thereof.

FIG. 3B shows side view of the emitter coil housing 303 comprising various different shapes of coils 304-310 that may be arranged inside said housing 303. In general, the most basic shapes of coils that may be used to emit homogeneous and inhomogeneous time-varying or stationary electromagnetic fields are selected from the group including solenoid/Tesla coil 304, current-carrying loop 305, current loop 306, Helmholtz coils 307, Maxwell coil 308, solenoid cone 309, variations on the current-carrying loop 310, and combinations thereof as well as other coil types not listed previously, including different geometrical shapes of the aforementioned coils.

Each of the above-mentioned coils 304-310 may be made out of spray insulated copper wires, or wires of different material, depending on the type of treatment protocol the patient will be receiving. Each of the selected coil 304-310, or combination thereof, is fixed inside of each emitter coil housing 303 to prevent its displacement within said housing 303 while placing each of the array of emitters 400 on the patient body.

Furthermore, in accordance with an embodiment of the present invention, the above-mentioned coils 304-310 may be made out of different metals, such as gold, silver, aluminium etc. and these may be changed based on the patient's need. For example, when a lighter set of coils 304-310 is needed because there is the array of emitters 400 placed on the patient body, aluminium coils might be used, and if a smaller number of the array of emitters 400 that are stronger is needed, gold coils may be used to maximize the efficiency of the electromagnetic field delivery, by minimizing resistance.

Therein is presented a general description of coils shapes but they may be further altered based on above-mentioned seven basic types, in order to optimize field strength and delivery treatment protocol based on disease type and duration of each treatment session.

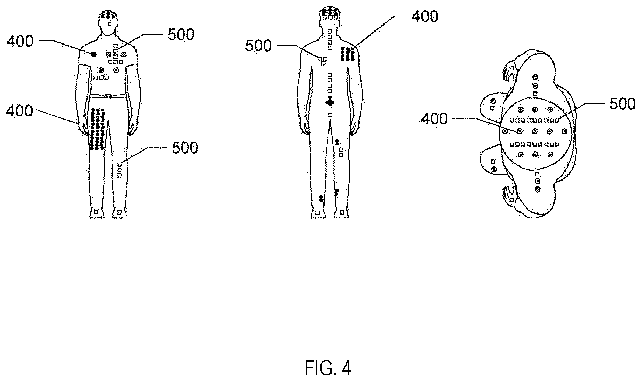

FIG. 4 illustrates front, back and top view of the most common variations in emitters 400 and sensors 500 positioning maps on the patient, depending on his underlying condition as well as tissue or organ affected by it. The patient's underlying condition, disease, disorder, or an indicated impaired functionality of the nervous system, organ or other tissue affected by injury, inflammation or ischemia determines the baseline treatment protocol, associated positioning map of the array of emitters 400 and, accordingly, the array of sensors 500. If the disease in question is neurological in nature, the array of emitters 400 may be placed along the patient's scalp 401, spinal cord 405 or other regions of the patient. In order to deliver the baseline (personalized) therapy protocol, i.e., personalized strength, frequency, nature and direction of electromagnetic fields within a certain time period, each of the array of sensors 500 for measuring electromagnetic fields, impedance, temperature and any other sensors, may be placed in a close proximity to each of the emitters 400, wherein each of the array of sensors 500 and each of the array of emitters 400 arranged in the close proximity to each other are operating as the pair. Other sensors may be placed on patient's heart and arms to monitor his vital functions.

As the portable apparatus 100 may be used for treatment of any condition, such as tissue affected by injury, inflammation or ischemia, from neurological to oncological, chronical, viral etc., the reference positioning map of the array of emitters 400 and sensors 500 and the baseline treatment protocol is established for each disease as well as organ or tissue separately. If the portable apparatus 100 is aimed to be used in magnetic, electric or electromagnetic guidance of implanted cells, injected or ingested drugs or medications, then the array of emitters 400 placement and their shape will greatly depend on the desired location which the therapy or implanted cells should reach.

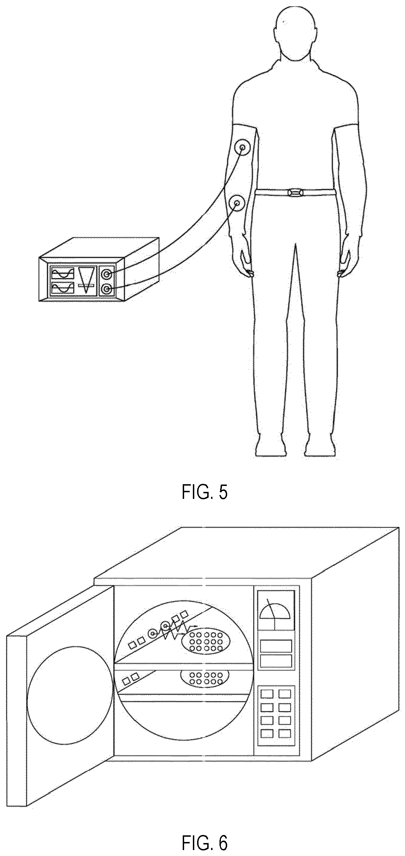

Figure 5 is another embodiment of the portable apparatus 100 illustrating an example therapy system that delivers electromagnetic field therapy to a patient to manage a disorder affected by viruses or infectious diseases in general. This embodiment of the portable apparatus 100 may be used to disable viral replication and reduce the efficiency of viral binding as well as other anti-pathogen mechanisms.

In general, when a virus is not inside a cell or is only beginning to infect the cell, it consists of three main parts: a genetic material that is responsible for encoding the structure proteins by which the virus acts, a capsid (protein coat) which protects the genetic material of the virus, and a viral envelope (phospholipid bilayer). On top of targeting the viral binding sites or deriving antigens for training of the immune system, like the vaccines that are currently in use, we also recognize three other potential approaches to combat the virus, once it is already in the organism, based on its construction, namely to: target the genetic material and disable viral replication, compromise the integrity of the capsid to expose the genetic material and compromise viral replication, and destroy the lipid envelope and disable viral binding.

Each of these approaches could be achieved using electromagnetic field, which can act on both the genetic and the lipid levels. For that purpose, the portable apparatus 100 may deliver electromagnetic-field-based anti-pathogen therapy for the systemic treatment of patients at specific frequencies and strengths that are non-harmful to host cells. When looking at viral suppression in living organisms, said therapy would have a two-fold action: targeting the viral genetic material and targeting the viral envelope. Targeting the viral genetic material comprises delivering of the inhomogeneous or homogeneous time-varying electromagnetic field frequencies, said frequencies would match up with the resonating frequency of the genetic material, impact its integrity and disable proper viral replication - slowing the progression of the virus and dampening its effects.

When looking at the phospholipid bilayer which makes up the lipid envelope of many viruses, as is the case with the novel SARS-CoV-2, it is a thin polar membrane which consists of a double layer of lipid molecules. The polarity of this lipid membrane makes it extremely electrically manipulatable due to its intrinsic electric dipole moment, leading to a charge separation to a positively and a negatively charged end.

One of the most interesting properties of such polarized lipid bilayers is flexoelectricity. This is a property of dielectric materials where they exhibit spontaneous electrical polarization that is induced by a strain gradient. This means that, once a mechanical stress is exerted on the virus, it exhibits electrical polarization. Because of their different structure and function, viruses should also pose a drastically different external surface charge from the innate cells of the immune system - making them potentially easily targetable using charge-specific field frequencies and strengths targeted to alter their structural integrity.

The portable apparatus 100 may impact on the viral envelope's conductivity, electric permittivity, magnetic permeability and its polarity. On top of that, due to the charged components on the lipid envelope, portable apparatus 100 changes the intrinsic polarization of the membrane. The currently recognized main mechanism by which SARS-CoV-2 infects a cell is through cleavage of the spike protein on the viral surface by proteins called TMPRSS2. This causes the release of the viral RNA into the cell, forcing the cell to produce copies of the virus that are disseminated to infect more cells. The portable apparatus 100 may be used to make the spike protein on the viral surface functionally unstable by altering their surface charge. In this way we may significantly reduce efficiency of the virus to enter human cells.

Figure 6 illustrates another embodiment of the portable apparatus 100 for use in a vaccine production. Application of electromagnetic-field-based apparatus may speed up the production of vaccine. Briefly, vaccine is a suspension of microorganisms that induces antibody production to protect against diseases. It relies highly on the organisms own immune system developing a proper immune response to the pathogen-delivered antigens and developing antibodies that would enable the body to fight against the pathogen once the host has been infected post vaccination application. The vaccines can be separated into two main types: a whole-agent vaccine and a subunit vaccine. The whole-agent vaccine can either be inactivated (killed) or attenuated (weakened) microorganism. As opposed to the inactivated vaccine where the microorganism is killed mostly using formalin, in attenuated vaccines, DNA/RNA mutations have accumulated during long-term cell culture growth and the microorganism is weakened. On the other hand, the subunit vaccine uses a part or a product of the microorganism and adjuvants (detergents or dead non-pathogenic bacteria) to increase the effectiveness of the vaccine. This vaccine includes antigens (or epitopes) that best stimulate the immune system, as is the case with Hepatitis B, pertussis or pneumonia caused by S. Pneumoniae vaccines. On top of these two major groups, there also exist conjugate vaccines, which are specialized subunit vaccines where antigens are linked to polysaccharides, this is also what makes them extremely complicated to make. These vaccines are most effective in the immature system of infants and they include H. Influenzae type B and pneumonia caused by S. Pneumoniae. There are also many experimental types of vaccines, two of which are DNA/RNA and recombinant vector vaccines. Whilst the DNA/RNA vaccines include the DNA/RNA of important antigens that are introduced to the cell, and these are being developed for influenza, herpes and HIV, the recombinant vector vaccines consist of an attenuated virus or bacterium (vector) used to introduce microbial DNA/RNA to cells. The recombinant vector vaccines are also tested for HIV, rabies and measles. Most common pneumonia or influenza vaccines currently used fall into either the subunit or the conjugate type vaccine. One of the major flaws of the subunit, conjugate and DNA/RNA vaccine production is that identifying specific antigen takes time - and this is specifically what is happening nowadays with the SARS-CoV-2 vaccine development. It is slowed down by the difficult and lengthy process of identifying specific antigens.

In order to facilitate better antigen retrieval and increase the speed of vaccine production, another embodiment of our electromagnetic system may be used to advance vaccine production in all stages: generation of an antigen, release and isolation of the antigen and purification. Before a vaccine is produced, the antigen that will trigger the organisms' immune response needs to be generated. This is done by harvesting and growing the pathogen's proteins or DNA/RNA by either: growing viruses on primary cells such as cells from chicken embryos, growing bacteria in bioreactors to optimize the production of the antigens and deriving recombinant proteins from the pathogen in yeast, bacteria or cell cultures. By performing generation of the antigen in the electromagnetic system of the present invention, the communication between immune cells is significantly accelerated through signaling pathway alteration or amplification, thus advancing the process of screening.

In order to release as much of the pathogen, the antigen is then separated from the cells and isolated from the proteins or any other parts of the growth medium that are still present. The electromagnetic system of the present invention speeds up and makes antigen separation more precise by generating a specific field gradient.

In order to purify the antigen, several separation steps are carried out, taking advantage of the differences in protein size, physio-chemical properties, binding affinity or biological activity. The electromagnetic system of the present invention is efficient in electromagnetic purification, taking advantage of the electromagnetic and electromechanical differences in proteins, speeding up the purification process and making it more efficient.

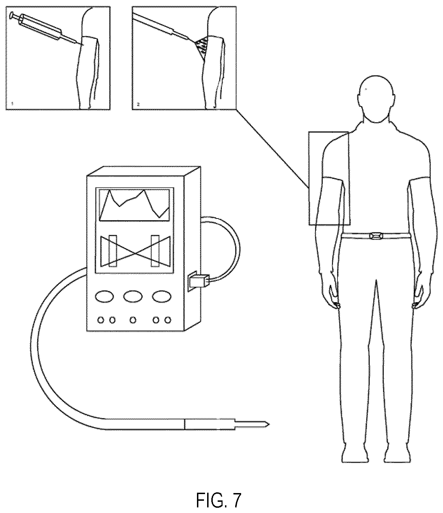

Figure 7 is another embodiment of the portable apparatus 100 illustrating an example therapy system that delivers electromagnetic field therapy to a patient to stimulate the immune system to respond to the vaccine more rapidly and vigorously. An adjuvant is added to enhance the recipient's immune response to the supplied antigen. After adding stabilizers or preservatives, all the components are then combined and mixed uniformly in a single vial. Because of potential incompatibilities and interactions between antigens and other ingredients, this step makes the development of combination vaccines much more challenging. Since, once an adjuvant is added, potential incompatibilities and unwanted interactions between antigens and other ingredients of the vaccine can occur, use of electromagnetic field therapy to the vaccine administered region will locally stimulate the immune system to respond to the vaccine more rapidly and vigorously.

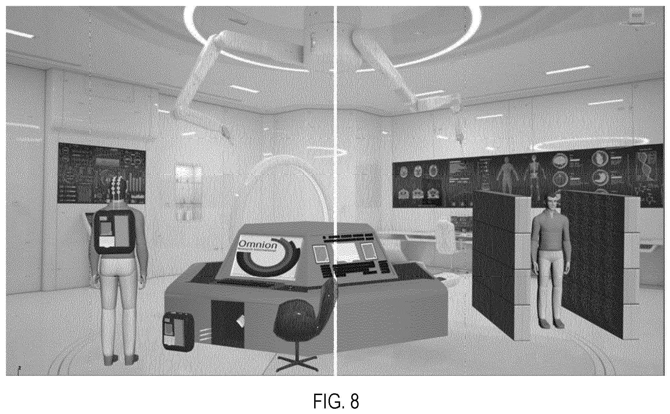

Figure 8 is another embodiment of the system of the present invention in a hospital environment. With the sudden worldwide recognized need to accelerate and improve drug production and vaccination not only for the new SARS-CoV-2 virus, but also for other viruses that are predicted to appear in the coming years, the system of the present invention may be modified as to a plurality of electrodes 400 emitting electromagnetic fields throughout the body to strengthen the immune system, either in response to new viruses or just as adjunctive therapy to patients undergoing chemotherapy, radiation or having a compromised immune system. As such, the system may be performed in a number of different ways for the purpose of treating acquired or congenital diseases of the organism or as a protection of healthy people. Although the main embodiment of the system is presented, it may also be performed in the form of the plurality of electrodes 400 on a wall surfaces that emit electromagnetic fields for the purpose of instant, but short-term, increase of a person's immunity. Its main benefits are that it offers systemic therapy and improves the overall functioning of the immune system and should be used by anyone at immediate risk of catching a virus or a bacterium (e.g., medical professionals, the elderly, the immunocompromised).

It should be understood with respect to any flowchart referenced herein that the division of the illustrated method into discrete operations represented by blocks of the flowchart has been selected for convenience and clarity only. Alternative division of the illustrated method into discrete operations is possible with equivalent results. Such alternative division of the illustrated method into discrete operations should be understood as representing other embodiments of the illustrated method.

Similarly, it should be understood that, unless indicated otherwise, the illustrated order of execution of the operations represented by blocks of any flowchart referenced herein has been selected for convenience and clarity only. Operations of the illustrated method may be executed in an alternative order, or concurrently, with equivalent results. Such reordering of operations of the illustrated method should be understood as representing other embodiments of the illustrated method.

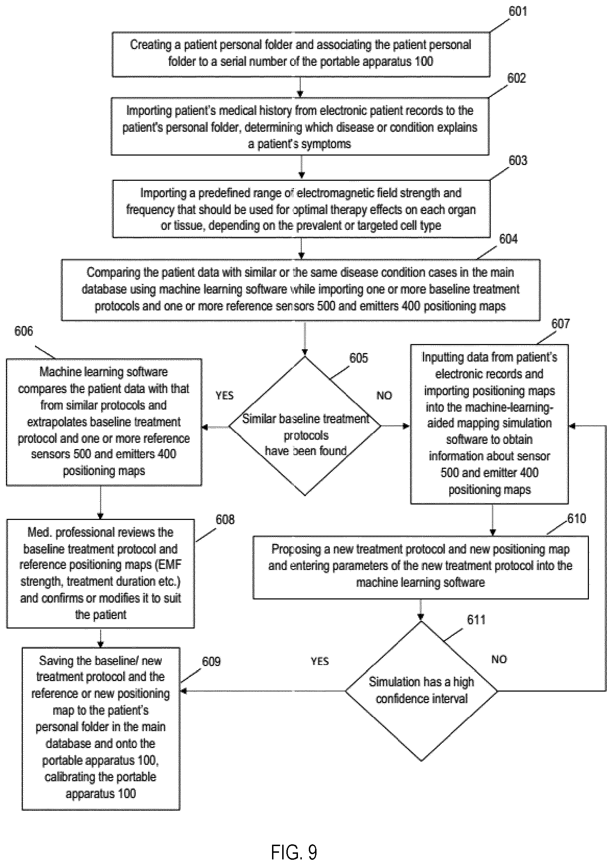

Figure 9 illustrates a flow chart of a method of generating an electromagnetic treatment protocol for treating the nervous system, other tissue or organ affected by injury, disease/disorder, inflammation or ischemia to a patient. In accordance with an embodiment of the present invention, the method of generating an electromagnetic treatment protocol for treating the nervous system other tissue or organ affected by injury, inflammation, disease/disorder or ischemia to a patient, the method including: creating a patient personal folder and associating the patient personal folder to a serial number of the portable apparatus 100 (block 601), importing patient's medical history (i.e. patient data) from electronic patient records to the patient's personal folder, determining which disease or condition explains a patient's symptoms and signs (block 602), importing a predefined electromagnetic field strength and frequency that should be used for optimal therapy effects on each organ or tissue, depending on the prevalent or targeted cell type (block 603), comparing by the machine learning software the patient data with similar or the same disease condition cases in a main database and importing one or more baseline treatment protocols and one or more reference sensors 500 and emitters 400 positioning maps (block 604), if similar baseline treatment protocols have been found (block 605) the machine learning software compares the patient data with that from similar protocols and extrapolates the baseline treatment protocol and one or more reference sensors 500 and emitters 400 positioning maps (block 606), reviewing by a medical professional the baseline treatment protocol and reference positioning maps (EMF strength, treatment duration, waveform, frequency etc.) and confirming or modifying the baseline treatment protocol into a new one to suite the patient (block 608) and saving the baseline/new treatment protocol and one reference or new positioning map to the patient's personal folder in the main database and onto the portable apparatus 100 and calibrating the portable apparatus 100 by a stationary device 200 to test the array of sensors 500 and emitters 400 for their accuracy and saving the baseline or the new treatment protocol in a storage space of the portable apparatus 100 before patient use (block 609). If similar baseline treatment protocols have not been found (block 605), imputing data from patient's electronic records and importing one or more positioning maps into an machine learning aided mapping simulation software to obtain information about sensor 500 and emitter 400 positioning map (block 607) and proposing a new treatment protocol and new sensor 500 and emitter 400 positioning map and entering parameters of the new treatment protocol into the machine learning software (block 610) and simulating of the new treatment protocol and new sensor 500 and emitter 400 positioning map to establish confidence interval (block 611). If a simulation does not have a high confidence interval, step at block 606 is repeated until high confidence interval has been accomplished, followed by saving the baseline/new treatment protocol and the new positioning map to the patient's personal folder, the main database and onto the portable apparatus 100, and calibrating the portable apparatus 100 by a stationary device 200 to test the array of sensors 500 and emitters 400 for their accuracy and saving the baseline or the new treatment protocol in a storage space of the portable apparatus 100 before patient use (block 609).

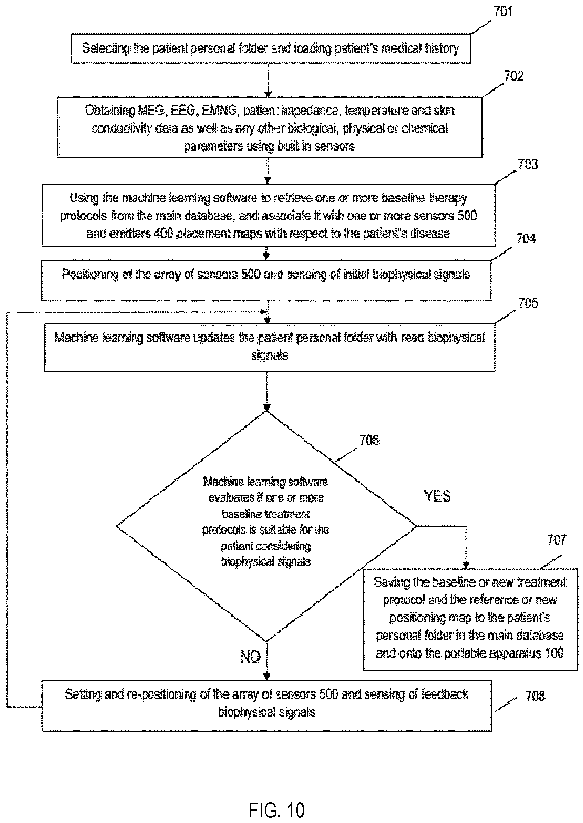

Fig. 10 is a flowchart depicting a method of generating an electromagnetic treatment protocol considering biophysical signals for treating the nervous system, other tissue or organ affected by injury, inflammation or ischemia and calibrating it to suit a patient's needs. Once the patient personal folder has been created and the medical history has been imported from the electronic patient records (blocks 601, 602, 701), and whilst still in the health institution, the baseline treatment protocol can be further optimized by taking additional readings of biophysical parameters, so that a machine learning software may propose the reference sensors 500 and emitters 400 positioning map on the patient's body. In order to obtain a more adequate baseline treatment protocol for the patient's disease or condition, the method of generating the baseline treatment protocol further includes obtaining MEG, EEG, EMNG, tissue impedance, temperature and skin conductivity as well as any other biological, physical or chemical parameters using built in sensors (block 702) or other diagnostic tools. After importing all of obtained parameters into the patient's electronic record, follows positioning of the array of sensors 500 and sensing of initial biophysical signals (block 704), the machine learning software updates the patient's personal folder with sensed initial biophysical signals (block 705) followed by running the machine learning software for retrieving from the main database one or more baseline therapy protocols and associated with one or more sensors 500 and emitters 400 placement maps associated with the patient's disease or condition (blocks 604, 703). After obtaining one or more sensors 500 and emitters 400 placement maps associated with the patient's disease or condition follows positioning of the array of sensors 500 and sensing of initial biophysical signals, wherein said signals include measurements of one or more types of neural activity, temperature, tissue impedance and electromagnetic fields (block 704). Afterwards, the machine learning software updates the patient personal folder with read biophysical signals (blocks 705) and evaluates if one or more baseline treatment protocols is suitable for the patient considering biophysical signals (block 706). If one of the baseline treatment protocols is suitable for the patient considering biophysical signals, the machine learning software is saving the baseline treatment protocol and the reference positioning map to the patient's personal folder in the main database and onto the portable apparatus 100 (blocks, 609 707). If one of the baseline treatment protocols is not suitable for the patient considering biophysical signals, the method further includes setting and re-positioning of the array of sensors 500 and sensing of biophysical signals (block 708) and providing feedback biophysical signals to machine learning software (block 705) in order to evaluate if one or more baseline treatment protocols is suitable for the patient considering biophysical signal feedback. If one of the baseline treatment protocols is suitable for the patient considering biophysical signal feedback, the machine learning software is saving a new treatment protocol and a new positioning map to the patient's personal folder in the main database and onto the portable apparatus 100 (blocks, 609 and 707). Re-positioning of the array of sensors 500 and sensing of feedback biophysical signals is repeated until the treatment protocol has achieved an intended effect. The new, personalized treatment protocol and associated re-positioning of the array of sensors 500 is personalized for each patient and may be stored in the main database as the baseline treatment protocol and the reference positioning map for use for other patients having similar disease and condition.

In order to prepare the portable apparatus 100 for using and determining an optimal baseline or new treatment protocol, the portable apparatus 100 is, via the connection port 204, connected to the stationary device 200 and the patient personal folder is created. The medical professional asks the patient about detailed information regarding his medical history, while the portable apparatus 100 is being calibrated and performs copying a patient data from online databases as well as allows for a manual input. The patient data is medical information held about an individual patient. The patient data may include information relating to their past and current health or illness, their treatment history, lifestyle choices and genetic data. It may also include biometric data, which is any measurable physical characteristic that can be checked by a machine/computer. In parallel, the machine learning software calculates the baseline treatment protocol for the patient, based on his medical data, by retrieving from the main database of treatment protocols comprising previously built therapy regime protocols developed during preclinical and clinical testing. This step determines a treatment duration, strength and nature of electromagnetic fields that are needed to be applied as well as suggests potential coil shapes to be used. Next, the machine learning software proceeds with comparing a recorded patient data with other patient cases with the goal of optimizing one or more baseline treatment protocols associated with the patient's disease. Said professional takes into account the information obtained from the patient, his medical record, and MEG, EEG, EMNG, tissue impedance, temperature and skin conductivity as well as any other biological, physical or chemical parameters using built in sensors and combines it into a new (personalized) treatment protocol taking into account one or more baseline treatment protocols the machine learning software proposition for therapy. Subsequently combined, the new treatment protocol is saved to the patient's portable apparatus 100 and in the personal patient folder of the stationary device 200, which enables the medical professional to view (and potentially adjustment) the ideal usage of the portable apparatus 100, while at the same time ensuring the maximal efficiency during treatments.

By taking into account additional information about patient's lifestyle choices (if the patient smokes, drinks too much alcohol, doesn't exercise enough etc.), the machine learning software can also make a calculation (based on a simulation) on which organ is suffering the most and what problems could the patient be presented with in the near future. This embodiment would be used in the predicting the treatment of EMF therapy.

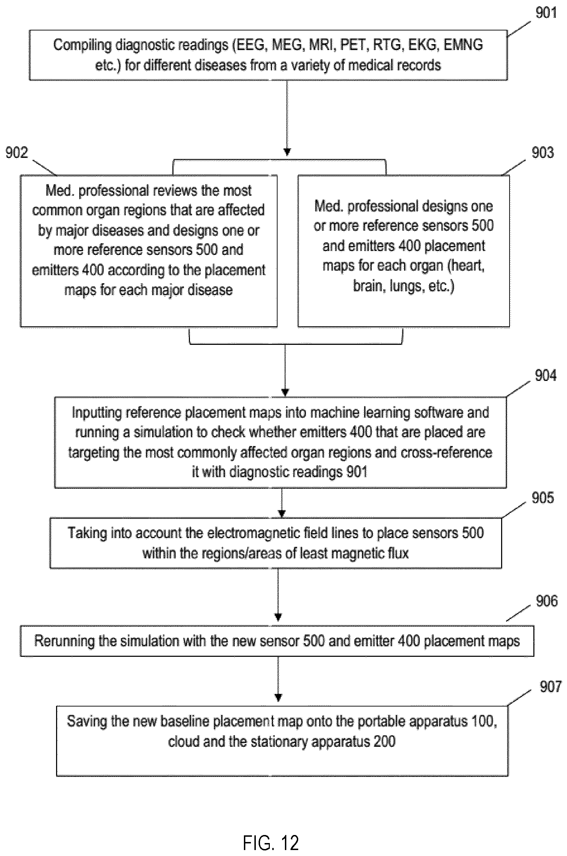

Fig. 12 is a flowchart depicting a method of generating one or more reference sensors 500 and emitters 400 placement maps, in accordance with an embodiment of the present invention.

The method of generating one or more reference sensors 500 and emitters 400 placement maps comprising the steps of: compiling diagnostic readings (EEG, MEG, MRI, PET, RTG, EKG, EMNG etc.) from a variety of medical records for different diseases within the major disease groups; reviewing by medical professional the most common organ regions that are affected by major diseases and designing one or more reference sensors 500 and emitters 400 placement maps for each major disease (block 902) and designing of one or more reference sensors 500 and emitters 400 placement maps for each organ (heart, brain, lungs, etc.) (block 903); inputting reference placement maps into machine learning software and running a simulation to check whether emitters 400 placement targets the most commonly affected organ regions and cross-referencing it with diagnostic readings 901 (block 904); placing sensors 500 within the regions/areas of least magnetic flux by taking into account the electromagnetic field lines (block 905); rerunning a machine learning aided software a simulation with new sensors 500 and emitters 400 placement map (block 906) wherein the simulation is run with a specific, predefined reference electromagnetic field range for providing optimal therapy effects on each organ or tissue, depending on the prevalent or targeted cell type that has been defined throughout preclinical and clinical testing and saving of the new baseline placement map onto the portable apparatus 100, cloud and the stationary apparatus 200 (block 907). The simulation is run with a specific, predefined electromagnetic field range for providing optimal therapy effects on each organ or tissue, depending on the prevalent or targeted cell type that has been defined throughout preclinical and clinical testing.

The major diseases and disorders include, but are not limited to, infectious diseases, deficiency diseases, hereditary diseases (including both genetic diseases and non-genetic hereditary diseases), and physiological diseases, which may be further divided based on the anatomic region impacted by those diseases/disorders into neural diseases, heart diseases, lung diseases, diseases of the digestive tract etc.

Once personalized sensors 500 and emitters 400 placement map is designed, the next step is for the array of sensors 500 to be placed on the patient's body, as per the machine learning software or previously designed general schemes/maps obtained during preclinical or clinical testing of the portable apparatus 100. The array of sensors 500 may now take initial readings about the tissue impedance, temperature and skin conductivity as well as any other biological, physical or chemical parameters using built in sensors. With that information the machine learning software updates the patient profile and checks if the proposed therapy is suited for the patient considering measured body parameters.

If the baseline treatment protocol is suitable, the portable apparatus 100 has been successfully calibrated and the patient may now take the portable apparatus 100 with him for home and everyday use, depending on the instructions from the medical professional. On the other hand, if the machine learning software suggests that the proposed therapy is unsuitable, it will be modified by repositioning the array of sensors 500, taking another round of measuring of biophysical signals and running machine learning software in order to update the therapy protocol. Then, if it is suitable, the patient can start the personalized treatment protocol.

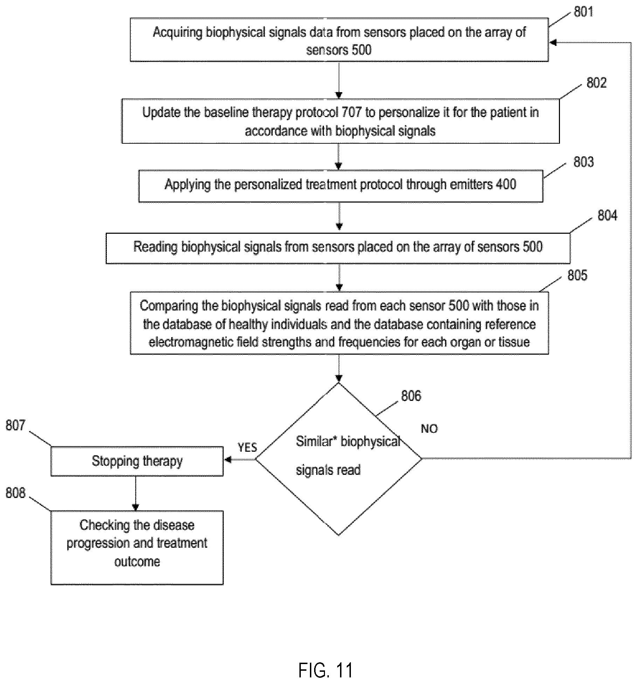

Fig. 11 is a flowchart depicting a method of treating disorders and diseases of the nervous system, other tissue or any organ affected by injury, inflammation or ischemia in an individual, in accordance with an embodiment of the present invention.

The method of treating disorders and diseases of the nervous system, other tissue or any organ affected by injury, inflammation or ischemia in an individual comprising: obtaining of biophysical signals from the array of sensors 500 (block 801); updating the baseline therapy protocol 707 to personalize it for the patient in accordance with read biophysical signals, applying the updated personalized treatment protocol in a real time through emitters 400 (block 803) and concurrently obtaining of biophysical signals from the array of sensors 500 (block 804), wherein each particular emitter 400 is positioned in a close proximity to each particular sensor 500, said emitter 400 and sensor 500 are operating as a pair; comparing the biophysical signals of each sensor 500, including but not limited to the reference electromagnetic field range, with those in the database of healthy individuals and the database containing reference electromagnetic field strengths and frequencies for each organ or tissue (block 805); wherein if similar biophysical signals are obtained for all or some of the array of sensors 500, stopping treatment protocol for all or some of the array of sensors 500 (block 807) and evaluating of disease progression and treatment outcome (block 808). On the other hand, if similar biophysical signals are not obtained for all or some of the array of sensors 500 (block 806) the retrieval of biophysical signals from the array of sensors 500 is repeated and steps 802 to 806 are repeated until all of the array of sensors 500 read electromagnetic fields that are within the reference electromagnetic field range, or, irrespective of obtained biophysical signals, until a specified duration of exposure to the therapeutic electromagnetic field defined by the baseline treatment protocol i.e. therapy session. Similar biophysical signals may be MEG, EEG, MRI, PET, RTG, EKG, EMNG etc., or similar medical history/diagnosis or similar symptoms.

The present invention provides a protocol generation and treatment system for use in treating physical diseases, mental diseases, infectious diseases, non- infectious diseases, deficiency diseases, inherited diseases, degenerative diseases etc.

The protocol generation and treatment system as described herein may be used in treating disorders and diseases of the nervous system, cognitive disorders such as epilepsy, bipolar disorders, schizophrenia, dementia, heart disorders, immunological or autoimmune disorders, tumors, cancer and other oncological disorders, viral or bacterial infections, inflammatory disorders, disorders and diseases of the muscle tissue and disorders and diseases of the connective tissue.

Neurological disorders are diseases of the central and peripheral nervous system. In other words, the brain, spinal cord, cranial nerves, peripheral nerves, nerve roots, autonomic nervous system, neuromuscular junction, and muscles.

The protocol generation and treatment system as described herein may be used in treating disorders and diseases of the nervous system including but not limited to epilepsy, Alzheimer disease and other dementias, cerebrovascular diseases including stroke, migraine and other headache disorders, multiple sclerosis, Parkinson's disease, Huntington's disease, ALS, neuro-infections, brain tumors (glioblastoma, astrocytoma, ependymoma etc.), traumatic disorders of the nervous system due to head trauma, and neurological disorders as a result of malnutrition. Moreover, they also include brain aneurysm, subdural/epidural hematoma, cerebral edema, stroke, physical brain injury, hydrocephalus, epilepsy and other neurological diseases and disorders.

The protocol generation and treatment system as described herein may be used in treating, without limitation, disorders and diseases selected from the group consisting of Vasculitis, Lupus, Cancer, Muscle spasms, Heart attack, Sports injuries, Muscular dystrophy, Cerebral palsy, Dermatomyositis, Compartment syndrome, Myasthenia gravis, Mitochondrial myopathies, Rhabdomyolysis, Polymyositis, Fibromyalgia, Myontonia, Myofascial pain syndrome, Muscle cramps, Sprains and strains, Tendinitis, Depression, Urinary and fecal incontinence, Hypertension, Back pain, Restless leg syndrome, Guillain Barre syndrome, Quadriplegia, Paraplegia, Diabetic polyneuropathy, Dyskinesias, Parethesias, Dental procedure pain, Knee osteoarthritis, Anesthesia, Angina, Ankylosing spondylitis, Burn pain, Cancer pain, Chronic pain, Dysmenorrhea, Headache, Hemiplegia, Hemiparesis, Labor pain, Facial pain, Trigeminal neuralgia, Bruxism pain, Myofascial pain, Pregnancy related nausea, Neck or shoulder pan, Pain from broken bones, Rib fracture, Diabetic peripheral neuropathy, Phantom limb pain, Post herpetic neuralgia, Postoperative ileus, Irritable bowel syndrome, Postoperative nausea or vomiting, Postoperative pain, Poststroke rehabilitation, Rheumatoid arthritis, Skin ulcers, Spinal cord injury, Temporomandibular joint pain, Detrusor instability, Spinal muscle atrophy(children), Pain during hysteroscopy, Gastroparesis, Chronic obstructive pulmonary disease rehabilitation, Carpal tunnel syndrome, Soft tissue injury, Intermittent claudication, Attention-deficit hyperactivity disorder (ADHD), Cognitive impairment, Knee replacement injury, Achalasia, Atrophic eczema, Bursitis, Dementia, Depression, Dry mouth dystonia, Enhanced blood flow in the brain, Enhanced blood perfusion of the uterus and placenta, Esophageal spasm, Fibromyalgia, Fracture pain, Guillain-Barre syndrome, Hemophilia, Herpes, Hip pain, Interstitial cystitis, Irritable bowel syndrome, Pruritis, Labor induction, Menstrual cramps, Muscle cramps, Muscle spasticity, Muscle strain or pain, Musculoskeletal trauma, Myofascial pain dysfunction syndrome, Nerve damage, Osteoarthritis, Pain medication adjunct, Pancreatitis, Raynaud's phenomenon, Repetitive strain injuries, Sacral pain, Shingles, Shoulder subluxation, Sickle cell anemia pain, Skin flap ischemia (plastic surgery), Sphincter of Oddi disorders, Sports injuries, Thrombophlebitis, Tinnitus, Tremor, Whiplash, Neuralgias, Sleep deprivation, Anxiety, Hallucinating, Migraine headaches, Posttraumatic stress disorder (PTSD), Phobic disorder, Borderline personality disorder, Appetite disorder, Fatigue, Impulse-control problems, Irritability, Mood problems, Movement problems, Tourette's syndrome, Trichotillomania, Violent/self-destructive behaviors, Allodynia, Decreasing the recovery period after nerve fiber damage or trauma, Tumors, Ischemic heart disease or coronary artery disease, Lower respiratory infections, Chronic obstructive pulmonary disease etc.

### EXAMPLES

### Example 1

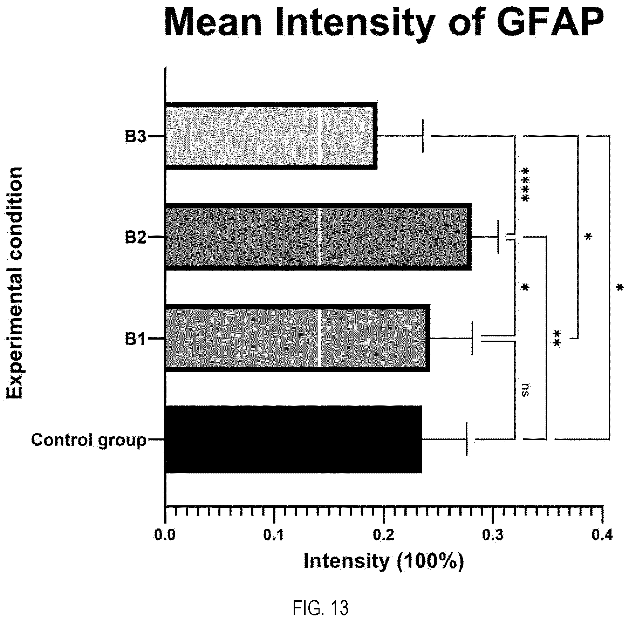

This example experiment, designed to assess the EMF effect on activity of microglia, astrocytes and oligodendrocytes, was performed on mixed glial cells isolated from C57BL6 albino mice, wherein results are shown in a diagram illustrating a mean intensity of glial fibrillary acidic protein in an experimental condition (see Fig. 13). Cells were plated at 100,000 cells/15.6 mm wells of a 24-well-plate in Dulbecco's Modified Eagle's Medium/Nutrient Mixture F-12 (DMEM/F-12) containing 10% heat-inactivated fetal bovine serum (FBS) and 1% Penicillin-Streptomycin (Pen Strep) and were allowed to stabilize for 5 days. Thereafter, the medium was replaced with 1% Pen Strep and DMEM/F-12 and the cells were allowed to stabilize at 37°C. An EMF apparatus was then placed into an incubator and cells were treated for 3 h with a non-thermal EMF signal, configured according to the teachings of this application, which consisted of a 1 - 6 A oscillating carrier pulse. This method was repeated for two additional EMF strengths, corresponding to 6 - 11 A and 11 - 15 A oscillating current strengths, including use of different coil shapes as well as changing nature of the fields. Cultures assigned to control groups were kept in a separate incubator at all times and were exposed to the same conditions in the absence of EMF signals. Afterwards, cells were fixed, immunocytochemically labeled with glial fibrillary acidic protein (GFAP) and cluster of differentiation molecule 11b (CD11b) and photographed for subsequent analysis. Cell nuclei were labeled with 4',6-diamidino-2-phenylindole (DAPI). Data was analyzed with ANOVA and a graphical depiction of the intensity of each labeling, expressed as a percentage of the total image intensity (100%), was made. In order to quantify the probability that an observed difference could have occurred just by random chance, P-value was used, where P ≤ 0.05 was considered statistically significant (*), P ≤ 0.01 very statistically significant (**), P ≤ 0.001 extremely statistically significant (***), P ≤ 0.0001 extremely statistically significant (****) and ns not statistically significant. The results in Fig. 13. show that EMF application significantly increased the cell activity, where a statistical significance can be seen between the control group and field intensity B2 (P ≤ 0.01) and field intensity B3 (P ≤ 0.05). Additional statistically significant differences can be seen between all three treatment groups where P ≤ 0.05 for B1 - B2 and B1 - B3, while P ≤ 0.0001 for B2 - B3. These results demonstrate that an EMF signal configured according to the present invention may modulate astrocytic, microglial and oligodendrocytic activity which, in turn, modulates cognitive processes, the immunological response as well as tissue repair and regeneration.

As the most abundant cells within the nervous system, astrocytes, together with microglia, are the crucial players of the neuroimmune system. While microglia function as the first stage of defense against foreign pathogens, astrocytes are involved in repair and regeneration of the injured tissue. Microglia also regulate the innate immune function of astrocytes, thus determining their neuroprotective or neurotoxic function. In turn, astrocytes secrete molecules that trigger microglial activation and regulate microglial phenotypes and function by impacting their motility and phagocytosis. Although astrocytes have so far only been regarded as neuronal supportive cells, assisting with CNS homeostasis regulation, various studies indicate astrocytes' role in the regulation of microglial phenotypes and functions, as well as the innate immune response within the CNS - suggesting that modulation of the astrocytic response could further regulate the microglial response.

As much as astrocytes play an indispensable role in physiological conditions and the immune response within the CNS, they also release neurotrophic factors such as transforming growth factor beta (TGF-β) and nerve growth factor (NGF), which play a role in the formation of the glial scar. Even though it was previously thought that the glial scar hinders axonal regeneration, recent studies have shown that the ablation of chronic astrocytic scars disabled the spontaneous regrowth of transected axons in spinal cord injury (SCI) lesions and increased axonal dieback. Thus, contrary to the accepted dogma, the formation of an astrocytic scar is crucial to axon regrowth and is a pivotal step in neurorestoration. Interpreting these results would suggest that, since different treatment modalities, as compared to the control group, impact astrocytic activity in distinct manners, this treatment could be used to:
I. Increase astrocytic activity
   This could cause further microglial activation and formation of an astrocytic scar - leading to neuroregeneration and neurorestoration, or
II. Decrease astrocytic activity
   This would be especially useful in diseases where the response of the innate immune system within the CNS is overactive (such as MS). Using this treatment modality, the astrocytic activity could be inhibited, leading to prevention of further demyelination, degeneration or autoimmune response.

### Example 2

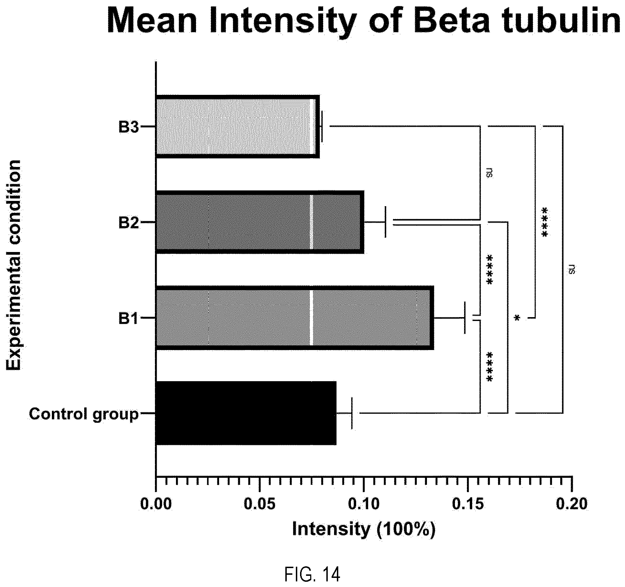

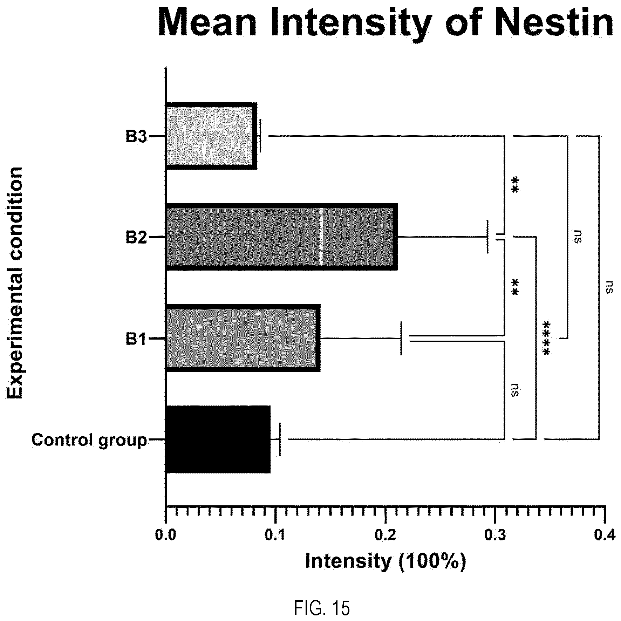

This example experiment, designed to assess the EMF effect on neuronal and NSC cell activity, was performed on cells isolated from C57BL6 albino mice, wherein results are shown in a diagram illustrating a mean intensity of beta tubulin in an experimental condition (see Fig. 14) and in a diagram illustrating a mean intensity of nestin in an experimental condition (see Fig. 15). Cells were plated at 50,000 cells/15.6 mm wells of a 24-well-plate in Dulbecco's Modified Eagle's Medium/Nutrient Mixture F-12 (DMEM/F-12) containing 10% heat-inactivated fetal bovine serum (FBS) and 1% Penicillin-Streptomycin (Pen Strep) and were allowed to stabilize for 5 days. Thereafter, the medium was replaced with 1% Pen Strep and DMEM/F-12 and the cells were allowed to stabilize at 37°C. An EMF apparatus was then placed into an incubator and cells were treated for 3 h with a non-thermal EMF signal, configured according to the teachings of this application, which consisted of a 1 - 6 A oscillating carrier pulse. This method was repeated for two additional EMF strengths, corresponding to 6 - 11 A and 11 - 15 A oscillating current strengths, including use of different coil shapes as well as changing the nature of the field and its frequency. Cultures assigned to control groups were kept in a separate incubator at all times and were exposed to the same conditions in the absence of EMF signals. Afterwards, cells were fixed, immunocytochemically labeled with β-tubulin (neurons) and nestin (NSC), and photographed for subsequent analysis. Cell nuclei were labeled with 4',6-diamidino-2-phenylindole (DAPI). Data was analyzed with ANOVA and a graphical depiction of the intensity of each labeling, expressed as a percentage of the total image intensity (100%), was made. In order to quantify the probability that an observed difference could have occurred just by random chance, P-value was used, where P ≤ 0.05 was considered statistically significant (*), P ≤ 0.01 very statistically significant (**), P ≤ 0.001 extremely statistically significant (***), P ≤ 0.0001 extremely statistically significant (****) and ns not statistically significant. The results in Fig. 14. show that EMF application significantly increased the neuronal activity, where a statistical significance can be seen between the control group and field intensity B1 (P ≤ 0.0001) and field intensity B2 (P ≤ 0.05). Additional statistically significant differences can be seen between the three treatment groups where P ≤ 0.0001 for B1 - B2 and B1 - B3. The results in Fig. 15. show that EMF application significantly increases the NSC activity, where a statistical significance can be seen between the control group and field intensity B2 (P ≤ 0.0001). Additional statistical significances can be seen between the treatment groups themselves where P ≤ 0.01 for B1 - B2 and B1 - B3. These results demonstrate that an EMF signal configured according to the present invention can modulate NSC and neuronal activity which, in turn, modulates cognitive processes, as well as neuronal repair and regeneration.

A neuron or a nerve cell is an electrically excitable cell that communicates with other cells via specialized connections called synapses. On the other hand, neural stem cells (NSCs) are self-renewing, multipotent cells that firstly generate the radial glial progenitor cells that generate the neurons and glia of the nervous system of all animals during embryonic development. Some neural progenitor stem cells persist in highly restricted regions in the adult vertebrate brain and continue to produce neurons throughout life.

With that in mind, interpreting our preliminary results would suggest that, since different treatment modalities, as compared to the control group, impact NSC and neuronal activity in distinct manners, this treatment could be used to:
I. Increase NSC/neuronal activity
   This could cause an increase in neuronal activity, strengthening the existing connections and potentially enabling the formation of new connections in diseases where those are impaired or lacking.
II. Decrease NSC/neuronal activity
   On the other hand, a certain treatment modality has shown a decrease in NSC/neuronal activity suggesting its potential use in inhibiting overactive neuronal connections in diseases with excessive neuronal firing.

Since NSCs, neurons, microglia, oligodendrocytes and astrocytes can be considered as the main building blocks of the nervous system and tissue, both central (CNS) and peripheral (PNS), we can assert the statement that our treatment modalities, which can be modified in intensity, strength and nature of the field to suit each patient, can indeed impact the nervous tissue and lead to disease treatment.

Additionally, since microglia are derived from embryonic mesoderm, which gives rise to cells of the blood and immune system, additional assertions can be made that a similar treatment protocol, with correspondence to the system of the present invention, can also be applied to the other systems of the body, including the immune system, for the purpose of disease and disorder treatment. In accordance with the former analogy, as neuronal processes naturally interweave with the myocytes resulting in a rich innervation of the myocytes, the system of the present invention could also be used for treatment of muscle diseases and disorders as well as other tissue that contains electrically excitable cells.

The invention is as defined by the appended claims.

## Claims

1. A protocol generation and treatment system comprising:
- a main database configured to store at least one patient personal folder comprising a medical patient data; a plurality of baseline treatment protocols, each baseline treatment protocol being associated with a particular disease or an indicated impaired functionality of the nervous system, organ or other tissue affected by injury, inflammation or ischemia; one or more associated reference sensors (500) and emitters (400) positioning maps; and representative neural tissue, organs and other tissue activity profiles associated with respective reference electromagnetic field ranges for each neural tissue, organ and cell type of healthy individuals
- a machine learning software and a processor configured to determine a disease or an indicated impaired functionality from the medical patient data, compare the medical patient data associated with the particular disease or the indicated impaired functionality with similar cases in the database, extrapolate one or more baseline treatment protocols associated with the particular disease or the indicated impaired functionality, to provide one or more reference sensors (500) and emitters (400) positioning maps associated to said baseline treatment protocols and to determine which of said positioning maps and baseline treatment protocols are suitable for the patient;
- a portable apparatus (100) comprising an electromagnetic field generator configured to generate electromagnetic fields; a storage space configured to save the baseline treatment protocol and reference sensors (500) and emitters (400) positioning map; an array of sensors (500) configured to measure biological, biophysical and biochemical signals including electromagnetic fields; an array of emitters (400) configured to deliver electromagnetic fields; a computing unit (104) configured to control the electromagnetic field generator, for recording in a real-time measured biophysical signal from each of the array of sensors (500), comparing each measured biophysical signal with the reference electromagnetic field range and to deliver a plurality of different electromagnetic fields through the array of emitters (400); and a power supply for components of the portable apparatus (100); and
- a stationary device (200) comprising the machine learning software, the processor and a communication interface, the stationary device (200) is configured to calibrate the portable apparatus (100), test the array of sensors (500) and emitters (400) for their accuracy, save the baseline treatment protocol and reference sensors (500) and emitters (400) positioning map in the main database and the storage space of the portable apparatus (100) before patient use, remotely control operation of the portable apparatus (100), and store information comprising data collected from an array of neural activity sensors placed on a patient during a session of applied stimuli and data collected from the array of sensors (500) that were placed on the patient before treatment application, wherein
- the computing unit (104) is further configured to modify electromagnetic fields delivered through each of the array of emitters (400) concurrently with measurement of biophysical signals from each of the array of sensors (500), wherein each of the array of emitters (400) and each of the array of sensors (500) positioned in a close proximity to each other are operating as a pair, and
- wherein the electromagnetic field delivered through each emitter (400) is continuously modified until it is achieved that the measured biophysical signal by a paired sensor (500) is being within the reference electromagnetic field range.

2. The system according to claim 1, wherein the reference range of electromagnetic field strength and frequency is provided for each organ or tissue based on a prevalent or targeted cell type.

3. The system according to claim 1, wherein the computing unit (104) is further configured to select one or more emitters (400) from the plurality of emitters (400) for excluding delivering the electromagnetic field based on the measured biophysical signal by one or more sensor (500) from the plurality of sensors (500) when said measured biophysical signal is being within the reference electromagnetic field range.

4. The system according to claim 1, wherein the machine learning software is further configured to modify the reference sensors (500) and emitters (400) positioning map and the baseline treatment protocol considering measured initial biophysical signals before the use of the portable apparatus (100).

5. The system according to claim 1, wherein each of the array of sensors (500) include tissue impedance sensors, temperature sensors, electric and magnetic field sensors, skin conductivity sensors, permittivity and permeability sensors and other biological, biochemical or biophysical sensors.

6. The system according to claim 1, wherein each of the array of emitters (400) include coils selected from the group consisting of solenoid/Tesla coil (304), current-carrying loop (305), current loop (306), Helmholtz coils (307), Maxwell coil (308), solenoid cone (309), variations on the current-carrying loop (310), or combinations thereof, including different geometrical shapes of the aforementioned coils.

7. The system according to claim 1, wherein the electromagnetic field delivered through each of the array of emitters (400) is a constant or time-varying homogenous and/or inhomogeneous electromagnetic field.

8. The system according to claim 1, wherein the reference electromagnetic field range is defined by its strength, direction, duration of application, waveform, frequency and amplitude for each organ, tissue and cell type.

9. The system according to claim 1, wherein to cause a destructive, partially destructive or constructive interference, the electromagnetic field delivered through each of the array of emitters (400) is **characterized by** a waveform being of an opposite or the same type to the one being measured by each paired sensor (500).

10. The system according to claim 9, wherein to cause a destructive, partially destructive or constructive interference, the electromagnetic field delivered through each of the array of emitters (400) is **characterized by** a waveform of a different or same amplitude or frequency to the one being measured by each paired sensor (500).

11. The system of any preceding claim, wherein the portable apparatus (100) includes a helmet, cap, or other headgear or arrangement for placement on or around the head of patient wherein the array of sensors (500) and the array of emitters (400) are arranged to be movable so that the electromagnetic field may be delivered to an area of brain affected by neurodegeneration, ischemia, injury or inflammation.

12. The system of any preceding claim, wherein the portable apparatus (100) includes a custom-made enclosure comprising an extender for attaching said closure to the patient and for treatment of any tissue or organ, wherein the machine learning software calculates the exact coordinates for an optimal position of the array of sensors (500) and emitters (400).

13. The system according to claim 12, wherein the custom-made enclosure is provided with a casing having adjustable pre-installed array of sensors (500) and emitters (400) which are movable along the X, Y and Z axis to position the array of sensors (500) and emitters (400) in accordance with the baseline treatment protocol and associated reference sensors (500) and emitters (400) positioning map.

14. The system of any preceding claim for use in treatment of disorders and diseases of the nervous system, cognitive disorders including epilepsy, bipolar disorders, schizophrenia, dementia, heart disorders, immunological or autoimmune disorders, tumors, cancer and other oncological disorders, viral or bacterial infections, inflammatory disorders, disorders, diseases of the muscle tissue and disorders and diseases of the connective tissue, and to stimulate an immune response after administering vaccines.

15. A portable apparatus (100) comprising an electromagnetic field generator configured to generate electromagnetic fields; an array of sensors (500) configured to measure biological, biophysical, and biochemical signals including electromagnetic fields; an array of emitters (400) configured to deliver electromagnetic fields; a storage space configured to store a baseline treatment protocol and a reference sensors (500) and emitters (400) positioning map; a computing unit (104) configured to control the electromagnetic field generator, for recording in a real-time measured biophysical signal from each of the array of sensors (500), comparing each measured biophysical signal with a reference electromagnetic field range and to deliver a plurality of different electromagnetic fields through the array of emitters (400); and a power supply for components of the portable apparatus (100), wherein the computing unit (104) is further configured to modify electromagnetic field delivered through each of the array of emitters (400) concurrently with measurement of the biophysical signal from each of the array of sensors (500), wherein each of the array of emitters (400) and each of the array of sensors (500) positioned in a close proximity to each other are operating as a pair, wherein the electromagnetic field delivered through each emitter (400) is continuously modified until it is achieved that the measured biophysical signal by a paired sensor (500) is being within the reference electromagnetic field range.

16. The portable apparatus (100) according to claim 15, wherein the computing unit (104) is further configured to select one or more emitter (400) from the plurality of emitters (400) for excluding delivering the electromagnetic field based on the measured biophysical signal by one or more sensor (500) from the plurality of sensors (500) when the measured biophysical is being within the reference electromagnetic field range.

17. The portable apparatus (100) according to claim 15, wherein each of the array of sensors (500) include tissue impedance sensors, temperature sensors, electric and magnetic field sensors, skin conductivity sensors, permittivity and permeability sensors or any combinations thereof.

18. The portable apparatus (100) according to claim 15, wherein each of the array of emitters (400) include coils selected from the group consisting of solenoid/Tesla coil (304), current-carrying loop (305), current loop (306), Helmholtz coils (307), Maxwell coil (308), solenoid cone (309), variations on the current-carrying loop (310), or combinations thereof, including different geometrical shapes of the aforementioned coils.

19. The portable apparatus (100) according to claim 15, wherein the electromagnetic field delivered through each of the array of emitters (400) is a constant or time-varying homogenous and/or inhomogeneous electromagnetic field.

20. The portable apparatus (100) according to claim 15, wherein the reference electromagnetic field range is defined by its strength, direction, duration of application, frequency and amplitude for each organ, tissue and cell type.

21. The portable apparatus (100) according to claim 15, wherein to cause a destructive, partially destructive or constructive interference, the electromagnetic field delivered through each of the array of emitters (400) is **characterized by** a waveform being of an opposite or the same type to the one being measured by the paired sensor (500).

22. The portable apparatus (100) according to claim 21, wherein to cause the destructive, partially destructive or constructive interference, the electromagnetic field delivered through each of the array of emitters (400) is **characterized by** the waveform of a different or same amplitude or frequency to the one being measured by the paired sensor (500).

23. The portable apparatus (100) according to any of claims 15 to 22, wherein further including a helmet, cap, or other headgear or arrangement for placement on or around the head of patient wherein the array of sensors (500) and the array of emitters (400) are arranged to be movable so that the electromagnetic field may be delivered to an area of brain affected by neurodegeneration, ischemia, injury or inflammation.

24. The portable apparatus (100) according to any of claims 15 to 22, wherein further including a custom-made enclosure comprising an extender for attaching said closure to the patient and for treatment of any tissue or organ, wherein the machine learning software calculates the exact coordinates for an optimal position of the array of sensors (500) and emitters (400).

25. The portable apparatus (100) according to claim 24, wherein the custom-made enclosure is provided with a casing having adjustable pre-installed array of sensors (500) and emitters (400) which are movable along the X, Y and Z axis to position the array of sensors (500) and emitters (400) in accordance with the baseline treatment protocol and associated reference sensors (500) and emitters (400) positioning map.

26. The portable apparatus according to claims 15-25 for use in treatment of disorders and diseases of the nervous system, cognitive disorders including epilepsy, bipolar disorders, schizophrenia, dementia, heart disorders, immunological or autoimmune disorders, tumors, cancer and other oncological disorders, viral or bacterial infections, inflammatory disorders, disorders, diseases of the muscle tissue and disorders, diseases of the connective tissue, and to stimulate an immune response after administering vaccines.

## Patentansprüche

1. System zur Generierung von Protokollen und Behandlung, umfassend:
- eine Hauptdatenbank, die dazu konfiguriert ist, mindestens einen persönlichen Patientenordner mit medizinischen Patientendaten zu speichern; eine Mehrzahl von Basis-Behandlungsprotokollen, wobei jedes Basis-Behandlungsprotokoll einer bestimmten Erkrankung oder einer angezeigten Beeinträchtigung der Funktionalität des Nervensystems, eines Organs oder eines anderen Gewebes zugeordnet ist, das durch Verletzung, Entzündung oder Ischämie betroffen ist; eine oder mehrere zugeordnete Referenz-Positionskarte für Sensoren (500) und Emitter (400); sowie repräsentative Aktivitätsprofile von neuronalen Geweben, Organen und anderen Geweben, die jeweils mit entsprechenden Referenzbereichen elektromagnetischer Felder für jedes neuronale Gewebe, jedes Organ und jeden Zelltyp gesunder Personen verbunden sind,
- eine Software für maschinelles Lernen und einen Prozessor, die dazu konfiguriert sind, aus den medizinischen Patientendaten eine Erkrankung oder eine angezeigte Funktionsbeeinträchtigung zu bestimmen, die medizinischen Patientendaten, die mit der jeweiligen Erkrankung oder der angezeigten Funktionsbeeinträchtigung in Verbindung stehen, mit ähnlichen Fällen in der Datenbank zu vergleichen, ein oder mehrere mit der jeweiligen Erkrankung oder der angezeigten Beeinträchtigung der Funktionalität verbundene Basis-Behandlungsprotokolle zu extrapolieren, eine oder mehrere den genannten Basis-Behandlungsprotokollen zugeordnete Referenz-Positionskarten für Sensoren (500) und Emitter (400) bereitzustellen, sowie zu bestimmen, welche der genannten Positionskarten und Basis-Behandlungsprotokolle für den Patienten geeignet sind;
- eine tragbare Vorrichtung (100), umfassend einen elektromagnetischen Feldgenerator, der zur Erzeugung elektromagnetischer Felder ausgelegt ist; eine Speichereinheit, die zur Speicherung des Basis-Behandlungsprotokolls sowie einer Referenz-Positionskarte der Sensoren (500) und Emitter (400) ausgelegt ist; eine Sensoranordnung (500), die zur Messung biologischer, biophysikalischer und biochemischer Signale einschließlich elektromagnetischer Felder ausgelegt ist; eine Ermitteranordnung (400), die zur Abgabe elektromagnetischer Felder ausgelegt ist; eine Recheneinheit (104), die zum Steuern des elektromagnetischen Feldgenerators, zum Aufzeichnen eines in Echtzeit gemessenen biophysikalischen Signals von jedem Sensor der Sensoranordnung (500), zum Vergleichen jedes gemessenen biophysikalischen Signals mit dem Referenzbereich des elektromagnetischen Felds und zum Abgabe einer Mehrzahl verschiedener elektromagnetischer Felder über die Emitteranordnung (400) konfiguriert ist; sowie eine Stromversorgung für die Komponenten der tragbaren Vorrichtung (100); und
- ein stationäres Gerät (200), umfassend eine Software für maschinelles Lernen, einen Prozessor sowie eine Kommunikationsschnittstelle, wobei das stationäre Gerät (200) dazu konfiguriert ist, die tragbare Vorrichtung (100) zu kalibrieren, die Sensoranordnung (500) und die Emitteranordnung (400) hinsichtlich ihrer Genauigkeit zu testen, das Basis-Behandlungsprotokoll sowie die Referenz-Positionskarte für Sensoren (500) und Emitter (400), vor der Anwendung am Patienten sowohl in der Hauptdatenbank als auch im Speicherbereich der tragbaren Vorrichtung (100) zu speichern, den Betrieb der tragbaren Vorrichtung (100) fernzusteuern, und Informationen zu speichern, die Daten umfassen, welche von einer Sensoranordnung zur Erfassung neuronaler Aktivität stammen, die während einer Sitzung angewandter Stimuli an einem Patienten angebracht ist, sowie Daten, die von der Sensoranordnung (500) erfasst wurden, die vor der Anwendung der Behandlung am Patienten angebracht war;
wobei
- die Recheneinheit (104) ferner dazu konfiguriert ist, elektromagnetische Felder, die von jedem Emitter der Emitteranordnung (400) abgegeben werden, gleichzeitig mit der Messung biophysikalischer Signale von jedem Sensor der Sensoranordnung (500) zu modifizieren, wobei jeder Emitter (400) und jeder Sensor (500), die in enger räumlicher Nähe zueinander positioniert sind, paarweise betrieben werden, und
- wobei das von jedem Emitter (400) abgegebene elektromagnetische Feld kontinuierlich modifiziert wird, bis erreicht ist, dass das von einem zugeordneten Sensor (500) gemessene biophysikalische Signal innerhalb des Referenzbereiches des elektromagnetischen Feldes liegt.

2. System nach Anspruch 1, wobei der Referenzbereich der elektromagnetischen Feldstärke und der Frequenz für jedes Organ oder Gewebe auf der Grundlage eines vorherrschenden oder gezielt ausgewählten Zelltyps bereitgestellt wird.

3. System nach Anspruch 1, wobei die Recheneinheit (104) ferner dazu konfiguriert ist, einen oder mehrere Emitter (400) aus der Mehrzahl von Emittern (400) auszuwählen, um die Abgabe des elektromagnetischen Feldes auf der Grundlage des gemessenen biophysikalischen Signals durch einen oder mehrere Sensoren (500) aus der Mehrzahl von Sensoren (500) auszuschließen, wenn das gemessene biophysikalische Signal innerhalb des Referenzbereichs des elektromagnetischen Feldes liegt.

4. System nach Anspruch 1, wobei die Software für maschinelles Lernen ferner dazu konfiguriert ist, die Referenz-Positionskarte für Sensoren (500) und Emitter (400) sowie das Basis-Behandlungsprotokoll unter Berücksichtigung gemessener initialer biophysikalischer Signale vor der Verwendung der tragbaren Vorrichtung (100) zu modifizieren.

5. System nach Anspruch 1, wobei jede Sensoranordnung (500) Gewebeimpedanzsensoren, Temperatursensoren, elektrische und magnetische Feldsensoren, Hautleitfähigkeitssensoren, Sensoren zur Erfassung der Permittivität und der Permeabilität sowie weitere biologische, biochemische oder biophysikalische Sensoren umfasst.

6. System nach Anspruch 1, wobei jede Emitteranordnung (400) Spulen umfasst, die aus der Gruppe ausgewählt sind, bestehend aus einer Solenoid-/Teslaspule (304), einer stromdurchflossenen Schleife (305), einer Stromschleife (306), Helmholtz-Spulen (307), einer Maxwell-Spule (308), einem kegelförmigen Solenoid (309), Variationen der stromdurchflossenen Schleife (310) oder Kombinationen davon, einschließlich unterschiedlicher geometrischer Formen der vorgenannten Spulen.

7. System nach Anspruch 1, wobei das von jedem Emitter der Emitteranordnung (400) abgegebene elektromagnetische Feld ein konstantes oder zeitlich veränderliches homogenes und/oder inhomogenes elektromagnetisches Feld ist.

8. System nach Anspruch 1, wobei der Referenzbereich des elektromagnetischen Feldes durch dessen Stärke, Richtung, Anwendungsdauer, Wellenform, Frequenz und Amplitude für jedes Organ, jedes Gewebe und jeden Zelltyp definiert ist.

9. System nach Anspruch 1, wobei zur Erzeugung einer destruktiven, teilweise destruktiven oder konstruktiven Interferenz das von jedem Emitter der Emitteranordnung (400) abgegebene elektromagnetische Feld durch eine Wellenform gekennzeichnet ist, die vom gleichen Typ oder von einem entgegengesetzten Typ wie diejenige ist, die von dem jeweils zugeordneten Sensor (500) gemessen wird.

10. System nach Anspruch 9, wobei zur Erzeugung einer destruktiven, teilweise destruktiven oder konstruktiven Interferenz das von jedem Emitter der Emitteranordnung (400) abgegebene elektromagnetische Feld durch eine Wellenform gekennzeichnet ist, deren Amplitude oder Frequenz gleich oder unterschiedlich zu derjenigen ist, die von dem zugeordneten Sensor (500) gemessen wird.

11. System nach einem der vorhergehenden Ansprüche, wobei die tragbare Vorrichtung (100) einen Helm, eine Kappe oder eine andere Kopfbedeckung oder Anordnung zur Anbringung auf oder um den Kopf des Patienten umfasst, wobei die Sensoranordnung (500) und die Emitteranordnung (400) beweglich angeordnet sind, sodass das elektromagnetische Feld an einen von Neurodegeneration, Ischämie, Verletzung oder Entzündung betroffenen Bereich des Gehirns abgegeben werden kann.

12. System nach einem der vorhergehenden Ansprüche, wobei die tragbare Vorrichtung (100) ein maßgefertigtes Gehäuse umfasst, das eine Verlängerung zur Befestigung dieses Gehäuses am Patienten und zur Behandlung von beliebigem Gewebe oder Organ aufweist, wobei die Software für maschinelles Lernen die exakten Koordinaten für eine optimale Position der Sensoranordnung (500) und der Emitteranordnung (400) berechnet.

13. System nach Anspruch 12, wobei das maßgefertigte Gehäuse mit einem Gehäusekörper versehen ist, der eine verstellbare, vorinstallierte Sensoranordnung (500) und Emitteranordnung (400) aufweist, wobei diese entlang der X-, Y- und Z-Achse beweglich sind, um die Sensoranordnung (500) und die Emitteranordnung (400) in Übereinstimmung mit dem Basis-Behandlungsprotokoll und der zugehörigen Referenz-Positionskarte für Sensoren (500) und Emitter (400) zu positionieren.

14. System nach einem der vorhergehenden Ansprüche zur Verwendung bei der Behandlung von Erkrankungen und Störungen des Nervensystems, kognitiven Störungen einschließlich Epilepsie, bipolaren Störungen, Schizophrenie und Demenz, Herzerkrankungen, immunologischen oder autoimmunen Erkrankungen, Tumoren, Krebs und anderen onkologischen Erkrankungen, viralen oder bakteriellen Infektionen, entzündlichen Erkrankungen, Erkrankungen und Störungen des Muskelgewebes sowie Erkrankungen des Bindegewebes und zur Stimulation einer Immunantwort nach der Verabreichung von Impfstoffen.

15. Tragbare Vorrichtung (100), umfassend einen elektromagnetischen Feldgenerator, der dazu konfiguriert ist, elektromagnetische Felder zu erzeugen; eine Sensoranordnung (500), die dazu konfiguriert ist, biologische, biophysikalische und biochemische Signale einschließlich elektromagnetischer Felder zu messen; eine Emitteranordnung (400), die dazu konfiguriert ist, elektromagnetische Felder abzugeben; einen Speicherplatz, der dazu konfiguriert ist, ein Basis-Behandlungsprotokoll und eine Referenz-Positionskarte für Sensoren (500) und Emitter (400) zu speichern; eine Recheneinheit (104), die dazu konfiguriert ist, den elektromagnetischen Feldgenerator zu steuern, in Echtzeit gemessene biophysikalische Signale von jedem Sensor (500) der Sensoranordnung aufzuzeichnen, jedes gemessene biophysikalische Signal mit einem Referenzbereich des elektromagnetischen Feldes zu vergleichen und mehrere unterschiedliche elektromagnetische Felder über die Emitteranordnung (400) abzugeben; und Stromversorgung für Komponenten der tragbaren Vorrichtung (100), wobei die Recheneinheit (104) ferner dazu konfiguriert ist, das elektromagnetische Feld, das von jedem Emitter der Emitteranordnung (400) abgegeben wird, gleichzeitig mit der Messung des biophysikalischen Signals von jedem Sensor der Sensoranordnung (500) zu modifizieren, wobei jeweils ein Emitter (400) der Emitteranordnung und ein Sensor (500) der Sensoranordnung, die in enger räumlicher Nähe zueinander angeordnet sind, als ein Paar betrieben werden und wobei das über jeden Emitter (400) abgegebene elektromagnetische Feld kontinuierlich modifiziert wird, bis das durch den jeweils zugeordneten Sensor (500) gemessene biophysikalische Signal innerhalb des Referenzbereichs des elektromagnetischen Feldes liegt.

16. Tragbare Vorrichtung (100) nach Anspruch 15, wobei die Recheneinheit (104) ferner dazu konfiguriert ist, einen oder mehrere Emitter (400) aus der Mehrzahl von Emittern (400) auszuwählen, um die Abgabe des elektromagnetischen Feldes auf der Grundlage des gemessenen biophysikalischen Signals durch einen oder mehrere Sensoren (500) aus der Mehrzahl von Sensoren (500) auszuschließen, wenn das gemessene biophysikalische Signal innerhalb des Referenzbereichs des elektromagnetischen Feldes liegt.

17. Tragbare Vorrichtung (100) nach Anspruch 15, wobei jede Sensoranordnung (500) Gewebeimpedanzsensoren, Temperatursensoren, elektrische und magnetische Feldsensoren, Hautleitfähigkeitssensoren, Sensoren zur Messung der Permittivität und der Permeabilität oder beliebige Kombinationen davon umfasst.

18. Tragbare Vorrichtung (100) nach Anspruch 15, wobei jede Emitteranordnung (400) Spulen umfasst, die aus der Gruppe ausgewählt sind, bestehend aus einer Solenoid-/Teslaspule (304), einer stromdurchflossenen Schleife (305), einer Stromschleife (306), Helmholtz-Spulen (307), einer Maxwell-Spule (308), einem kegelförmigen Solenoid (309), Variationen der stromdurchflossenen Schleife (310) oder Kombinationen davon, einschließlich unterschiedlicher geometrischer Formen der vorgenannten Spulen.

19. Tragbare Vorrichtung (100) nach Anspruch 15, wobei das von jedem Emitter der Emitteranordnung (400) abgegebene elektromagnetische Feld ein konstantes oder zeitlich veränderliches homogenes und/oder inhomogenes elektromagnetisches Feld ist.

20. Tragbare Vorrichtung (100) nach Anspruch 15, wobei der Referenzbereich des elektromagnetischen Feldes durch dessen Stärke, Richtung, Anwendungsdauer, Wellenform, Frequenz und Amplitude für jedes Organ, jedes Gewebe und jeden Zelltyp definiert ist.

21. Tragbare Vorrichtung (100) nach Anspruch 15, wobei zur Erzeugung einer destruktiven, teilweise destruktiven oder konstruktiven Interferenz das von jedem Emitter der Emitteranordnung (400) abgegebene elektromagnetische Feld durch eine Wellenform gekennzeichnet ist, die vom gleichen Typ oder von einem entgegengesetzten Typ wie diejenige ist, die von dem jeweils zugeordneten Sensor (500) gemessen wird.

22. Tragbare Vorrichtung (100) nach Anspruch 21, wobei zur Erzeugung der destruktiven, teilweise destruktiven oder konstruktiven Interferenz das von jedem Emitter der Emitteranordnung (400) abgegebene elektromagnetische Feld durch eine Wellenform gekennzeichnet ist, deren Amplitude oder Frequenz gleich oder unterschiedlich zu derjenigen ist, die von dem zugeordneten Sensor (500) gemessen wird.

23. Tragbare Vorrichtung (100) nach einem der Ansprüche 15 bis 22, ferner umfassend einen Helm, eine Kappe oder eine andere Kopfbedeckung oder Anordnung zur Anbringung auf oder um den Kopf des Patienten, wobei die Sensoranordnung (500) und die Emitteranordnung (400) beweglich angeordnet sind, sodass das elektromagnetische Feld an einen von Neurodegeneration, Ischämie, Verletzung oder Entzündung betroffenen Bereich des Gehirns abgegeben werden kann.

24. Tragbare Vorrichtung (100) nach einem der Ansprüche 15 bis 22, ferner umfassend ein maßgefertigtes Gehäuse, das eine Verlängerung zur Befestigung dieses Gehäuses am Patienten und zur Behandlung von beliebigem Gewebe oder Organ aufweist, wobei die Software für maschinelles Lernen die exakten Koordinaten für eine optimale Position der Sensoranordnung (500) und der Emitteranordnung (400) berechnet.

25. Tragbare Vorrichtung (100) nach Anspruch 24, wobei das maßgefertigte Gehäuse mit einem Gehäusekörper versehen ist, der eine verstellbare, vorinstallierte Sensoranordnung (500) und Emitteranordnung (400) aufweist, wobei diese entlang der X-, Y- und Z-Achse beweglich sind, um die Sensoranordnung (500) und die Emitteranordnung (400) in Übereinstimmung mit dem Basis-Behandlungsprotokoll und der zugehörigen Referenz-Positionskarte für Sensoren (500) und Emitter (400) zu positionieren.

26. Tragbare Vorrichtung nach einem der Ansprüche 15 bis 25, zur Verwendung bei der Behandlung von Erkrankungen und Störungen des Nervensystems, von kognitiven Störungen einschließlich Epilepsie, bipolaren Störungen, Schizophrenie und Demenz, Herzerkrankungen, immunologischen oder autoimmunen Erkrankungen, Tumoren, Krebs und anderen onkologischen Erkrankungen, viralen oder bakteriellen Infektionen, entzündlichen Erkrankungen, Erkrankungen und Störungen des Muskelgewebes sowie Erkrankungen des Bindegewebes und zur Stimulation einer Immunantwort nach der Verabreichung von Impfstoffen.

## Revendications

1. Système de génération et de traitement de protocoles comprenant :
- une base de données principale configurée pour stocker au moins un dossier personnel de patient comprenant des données médicales du patient ; une pluralité de protocoles de traitement de référence, chaque protocole de traitement de référence étant associé à une maladie particulière ou à une altération fonctionnelle indiquée du système nerveux, d'un organe ou d'un autre tissu affecté par une lésion, une inflammation ou une ischémie, à une ou plusieurs cartes de positionnement associées de capteurs de référence (500) et d'émetteurs (400), ainsi qu'à des profils d'activité représentatifs des tissus nerveux, des organes et d'autres tissus associés à des plages de référence de champs électromagnétiques respectives pour chaque type de tissu nerveux, d'organe et de cellule chez des individus sains ;
- un logiciel d'apprentissage automatique et un processeur configurés pour déterminer une maladie ou une altération fonctionnelle indiquée à partir des données médicales du patient, comparer les données médicales du patient associées à la maladie particulière ou à l'altération fonctionnelle indiquée avec des cas similaires dans la base de données, extrapoler un ou plusieurs protocoles de traitement de référence associés à la maladie particulière ou à l'altération fonctionnelle indiquée, fournir une ou plusieurs cartes de positionnement de capteurs de référence (500) et d'émetteurs (400) associées auxdits protocoles de traitement de référence, et déterminer lesquelles desdites cartes de positionnement et desdits protocoles de traitement de référence sont adaptées au patient ;
- un appareil portable (100) comprenant un générateur de champ électromagnétique configuré pour générer des champs électromagnétiques ; un espace de stockage configuré pour enregistrer le protocole de traitement de référence et la carte de positionnement des capteurs de référence (500) et des émetteurs (400) ; un ensemble de capteurs (500) configuré pour mesurer des signaux biologiques, biophysiques et biochimiques, y compris des champs électromagnétiques ; un ensemble d'émetteurs (400) configuré pour délivrer des champs électromagnétiques ; une unité de calcul (104) configurée pour contrôler le générateur de champ électromagnétique, pour enregistrer en temps réel un signal biophysique mesuré provenant de chacun des capteurs de l'ensemble de capteurs (500), comparer chaque signal biophysique mesuré à la plage de référence de champ électromagnétique et délivrer une pluralité de champs électromagnétiques différents au moyen de l'ensemble d'émetteurs (400) ; et une alimentation électrique pour les composants de l'appareil portable (100) ; et
- un dispositif stationnaire (200) comprenant le logiciel d'apprentissage automatique, le processeur et une interface de communication, le dispositif stationnaire (200) étant configuré pour étalonner l'appareil portable (100), tester l'ensemble de capteurs (500) et d'émetteurs (400) quant à leur précision, enregistrer le protocole de traitement de référence et la carte de positionnement des capteurs de référence (500) et des émetteurs (400) dans la base de données principale et dans l'espace de stockage de l'appareil **portable** (100) avant l'utilisation par le patient, commander à distance le fonctionnement de l'appareil portable (100), et stocker des informations comprenant des données collectées à partir d'un ensemble de capteurs d'activité neuronale placés sur un patient au cours d'une séance de stimuli appliqués et des données collectées à partir de l'ensemble de capteurs (500) qui ont été placés sur le patient avant l'application du traitement,
dans lequel
- l'unité de calcul (104) est en outre configurée pour modifier les champs électromagnétiques délivrés par chacun des émetteurs de l'ensemble d'émetteurs (400) conjointement à la mesure des signaux biophysiques provenant de chacun des capteurs de l'ensemble de capteurs (500), chaque émetteur (400) et chaque capteur (500) étant positionnés à proximité immédiate l'un de l'autre et fonctionnant en paire, et
- dans lequel le champ électromagnétique délivré par chaque émetteur (400) est modifié de manière continue jusqu'à ce que le signal biophysique mesuré par le capteur (500) apparié se situe dans la plage de référence de champ électromagnétique.

2. Système selon la revendication 1, dans lequel la plage de référence de l'intensité et de la fréquence du champ électromagnétique est fournie pour chaque organe ou tissu sur la base d'un type cellulaire prévalent ou ciblé.

3. Système selon la revendication 1, dans lequel l'unité de calcul (104) est en outre configurée pour sélectionner un ou plusieurs émetteurs (400) parmi la pluralité d'émetteurs (400) afin de les exclure de la délivrance du champ électromagnétique, sur la base du signal biophysique mesuré par un ou plusieurs capteurs (500) parmi la pluralité de capteurs (500), lorsque ledit signal biophysique mesuré se situe dans la plage de référence de champ électromagnétique.

4. Système selon la revendication 1, dans lequel le logiciel d'apprentissage automatique est en outre configuré pour modifier la carte de positionnement des capteurs de référence (500) et des émetteurs (400) ainsi que le protocole de traitement de référence, en tenant compte des signaux biophysiques initiaux mesurés avant l'utilisation de l'appareil portable (100).

5. Système selon la revendication 1, dans lequel chacun des capteurs de l'ensemble de capteurs (500) comprend des capteurs d'impédance tissulaire, des capteurs de température, des capteurs de champs électriques et magnétiques, des capteurs de conductivité cutanée, des capteurs de permittivité et de perméabilité, ainsi que d'autres capteurs biologiques, biochimiques ou biophysiques.

6. Système selon la revendication 1, dans lequel chacun des émetteurs de l'ensemble d'émetteurs (400) comprend des bobines sélectionnées dans le groupe constitué de : bobine solénoïde / bobine de Tesla (304), boucle parcourue par un courant (305), boucle de courant (306), bobines de Helmholtz (307), bobine de Maxwell (308), cône solénoïde (309), variantes de la boucle parcourue par un courant (310), ou des combinaisons de celles-ci, y compris différentes formes géométriques desdites bobines.

7. Système selon la revendication 1, dans lequel le champ électromagnétique délivré par chacun des émetteurs de l'ensemble d'émetteurs (400) est un champ électromagnétique homogène et/ou inhomogène, constant ou variable dans le temps.

8. Système selon la revendication 1, dans lequel la plage de référence de champ électromagnétique est définie par son intensité, sa direction, sa durée d'application, sa forme d'onde, sa fréquence et son amplitude pour chaque organe, tissu et type cellulaire.

9. Système selon la revendication 1, dans lequel, afin de provoquer une interférence destructive, partiellement destructive ou constructive, le champ électromagnétique délivré par chacun des émetteurs de l'ensemble d'émetteurs (400) est **caractérisé par** une forme d'onde étant du type opposé ou du même type que celle mesurée par chaque capteur (500) apparié.

10. Système selon la revendication 9, dans lequel, afin de provoquer une interférence destructive, partiellement destructive ou constructive, le champ électromagnétique délivré par chacun des émetteurs de l'ensemble d'émetteurs (400) est **caractérisé par** une forme d'onde d'une amplitude ou d'une fréquence différente ou identique à celle mesurée par chaque capteur (500) apparié.

11. Système selon l'une quelconque des revendications précédentes, dans lequel l'appareil portable (100) comprend un casque, une coiffe ou tout autre dispositif ou agencement de port sur ou autour de la tête du patient, dans lequel l'ensemble de capteurs (500) et l'ensemble d'émetteurs (400) sont agencés de manière mobile, de sorte que le champ électromagnétique puisse être délivré à une zone du cerveau affectée par une neurodégénérescence, une ischémie, une lésion ou une inflammation.

12. Système selon l'une quelconque des revendications précédentes, dans lequel l'appareil portable (100) comprend une enveloppe sur mesure comprenant une extension destinée à fixer ladite enveloppe au patient et à permettre le traitement de tout tissu ou organe, le logiciel d'apprentissage automatique calculant les coordonnées exactes pour une position optimale de l'ensemble de capteurs (500) et d'émetteurs (400).

13. Système selon la revendication 12, dans lequel l'enveloppe sur mesure est pourvue d'un boîtier comportant un ensemble de capteurs (500) et d'émetteurs (400) préinstallé et réglable, lesquels sont déplaçables selon les axes X, Y et Z afin de positionner l'ensemble de capteurs (500) et d'émetteurs (400) conformément au protocole de traitement de référence et à la carte de positionnement associée des capteurs de référence (500) et des émetteurs (400).

14. Système selon l'une quelconque des revendications précédentes, destiné à être utilisé dans le traitement de troubles et de maladies du système nerveux, de troubles cognitifs comprenant l'épilepsie, les troubles bipolaires, la schizophrénie, la démence, les affections cardiaques, les troubles immunologiques ou auto-immuns, les tumeurs, le cancer et autres affections oncologiques, les infections virales ou bactériennes, les affections inflammatoires, les troubles et maladies du tissu musculaire ainsi que les troubles et maladies du tissu conjonctif, et pour stimuler une réponse immunitaire après l'administration de vaccins.

15. **Appareil** portable (100) comprenant un générateur de champ électromagnétique configuré pour générer des champs électromagnétiques, un ensemble de capteurs (500) configuré pour mesurer des signaux biologiques, biophysiques et biochimiques, y compris des champs électromagnétiques, un ensemble d'émetteurs (400) configuré pour délivrer des champs électromagnétiques, un espace de stockage configuré pour stocker un protocole de traitement de référence et une carte de positionnement des capteurs de référence (500) et des émetteurs (400), une unité de calcul (104) configurée pour contrôler le générateur de champ électromagnétique, pour enregistrer en temps réel un signal biophysique mesuré provenant de chacun des capteurs de l'ensemble de capteurs (500), comparer chaque signal biophysique mesuré à une plage de référence de champ électromagnétique et délivrer une pluralité de champs électromagnétiques différents au moyen de l'ensemble d'émetteurs (400), ainsi qu'une alimentation électrique pour les composants de l'appareil portable (100), dans lequel l'unité de calcul (104) est en outre configurée pour modifier le champ électromagnétique délivré par chacun des émetteurs de l'ensemble d'émetteurs (400) conjointement à la mesure du signal biophysique provenant de chacun des capteurs de l'ensemble de capteurs (500), chaque émetteur (400) et chaque capteur (500) positionnés à proximité immédiate l'un de l'autre fonctionnant en paire, le champ électromagnétique délivré par chaque émetteur (400) étant modifié de manière continue jusqu'à ce que le signal biophysique mesuré par le capteur (500) apparié se situe dans la plage de référence de champ électromagnétique.

16. Appareil portable (100) selon la revendication 15, dans lequel l'unité de calcul (104) est en outre configurée pour sélectionner un ou plusieurs émetteurs (400) parmi la pluralité d'émetteurs (400) afin de les exclure de la délivrance du champ électromagnétique, sur la base du signal biophysique mesuré par un ou plusieurs capteurs (500) parmi la pluralité de capteurs (500), lorsque le signal biophysique mesuré se situe dans la plage de référence de champ électromagnétique.

17. Appareil portable (100) selon la revendication 15, dans lequel chacun des capteurs de l'ensemble de capteurs (500) comprend des capteurs d'impédance tissulaire, des capteurs de température, des capteurs de champs électriques et magnétiques, des capteurs de conductivité cutanée, des capteurs de permittivité et de perméabilité, ou toute combinaison de ceux-ci.

18. Appareil portable (100) selon la revendication 15, dans lequel chacun des émetteurs de l'ensemble d'émetteurs (400) comprend des bobines sélectionnées dans le groupe constitué de : bobine solénoïde / bobine de Tesla (304), boucle parcourue par un courant (305), boucle de courant (306), bobines de Helmholtz (307), bobine de Maxwell (308), cône solénoïde (309), variantes de la boucle parcourue par un courant (310), ou des combinaisons de celles-ci, y compris différentes formes géométriques desdites bobines.

19. Appareil portable (100) selon la revendication 15, dans lequel le champ électromagnétique délivré par chacun des émetteurs de l'ensemble d'émetteurs (400) est un champ électromagnétique homogène et/ou inhomogène, constant ou variable dans le temps.

20. Appareil portable (100) selon la revendication 15, dans lequel la plage de référence de champ électromagnétique est définie par son intensité, sa direction, sa durée d'application, sa fréquence et son amplitude pour chaque organe, tissu et type cellulaire.

21. Appareil portable (100) selon la revendication 15, dans lequel, afin de provoquer une interférence destructive, partiellement destructive ou constructive, le champ électromagnétique délivré par chacun des émetteurs de l'ensemble d'émetteurs (400) est **caractérisé par** une forme d'onde étant du type opposé ou du même type que celle mesurée par le capteur (500) apparié.

22. Appareil portable (100) selon la revendication 21, dans lequel, afin de provoquer une interférence destructive, partiellement destructive ou constructive, le champ électromagnétique délivré par chacun des émetteurs de l'ensemble d'émetteurs (400) est **caractérisé par** une forme d'onde d'une amplitude ou d'une fréquence différente ou identique à celle mesurée par le capteur (500) apparié.

23. Appareil portable (100) selon l'une quelconque des revendications 15 à 22, comprenant en outre un casque, une coiffe ou tout autre dispositif ou agencement destiné à être placé sur ou autour de la tête du patient, dans lequel l'ensemble de capteurs (500) et l'ensemble d'émetteurs (400) sont agencés de manière mobile, de sorte que le champ électromagnétique puisse être délivré à une zone du cerveau affectée par une neurodégénérescence, une ischémie, une lésion ou une inflammation.

24. Appareil portable (100) selon l'une quelconque des revendications 15 à 22, comprenant en outre une enveloppe sur mesure comprenant une extension destinée à fixer ladite enveloppe au patient et à permettre le traitement de tout tissu ou organe, le logiciel d'apprentissage automatique calculant les coordonnées exactes pour une position optimale de l'ensemble de capteurs (500) et d'émetteurs (400).

25. Appareil portable (100) selon la revendication 24, dans lequel l'enveloppe sur mesure est pourvue d'un boîtier comportant un ensemble de capteurs (500) et d'émetteurs (400) préinstallé et réglable, lesquels sont déplaçables selon les axes X, Y et Z afin de positionner l'ensemble de capteurs (500) et d'émetteurs (400) conformément au protocole de traitement de référence et à la carte de positionnement associée des capteurs de référence (500) et des émetteurs (400).

26. Appareil portable selon les revendications 15 à 25, destiné à être utilisé dans le traitement de troubles et de maladies du système nerveux, de troubles cognitifs comprenant l'épilepsie, les troubles bipolaires, la schizophrénie, la démence, les affections cardiaques, les troubles immunologiques ou auto-immuns, les tumeurs, le cancer et autres affections oncologiques, les infections virales ou bactériennes, les affections inflammatoires, les troubles et maladies du tissu musculaire ainsi que les troubles et maladies du tissu conjonctif, et pour stimuler une réponse immunitaire après l'administration de vaccins.
